# EUROPEAN PATENT APPLICATION

(11) **EP 3 838 291 A1**
(43) Date of publication of application: **23.06.2021**
(21) Application number: 19849381.9
(22) Date of filing: 13.08.2019
(51) Int. Cl.: A61K 45/00, A61K 31/7008, A61K 48/00, A61P 35/00

(54) **CANCER STEM CELL MARKER AND CANCER STEM CELL TARGETING DRUG**

(30) Priority: 13.08.2018 JP 2018152526
(71) Applicant: Cancerstem Tech Inc., Ibaraki-shi, Osaka 567-0085 (JP)
(72) Inventor: MORI, Masaki, Toyonaka-shi, Osaka 560-0082 (JP); YAMAMOTO, Hirofumi, Osaka-shi, Osaka 546-0032 (JP); WU, Xin, Nishinomiya-shi, Hyogo 663-8143 (JP)
(74) Representative: Heubeck, Christian
(86) International application number: PCT/JP2019/031894
(87) International publication number: WO 2020/036183

(57) **Abstract**

The present invention provides a cancer stem cell elimination agent containing a substance that suppresses an expression or function of SDC4, a method for detecting or sorting a cancer stem cell in a cancer cell population, including using an expression of SDC4 as an index, and the like.

SEQUENCE LISTING

<110>  CANCERSTEM TECH INC.

<120>  Marker for Cancer Stem Cell and Drug for Targeting Cancer Stem
       Cell

<130>  092921

<150>  JP 2018-152526
<151>  2018-08-13

<160>  16

<170>  PatentIn version 3.5

<210>  1
<211>  2615
<212>  DNA
<213>  Homo sapiens


<220>
<221>  CDS
<222>  (41)..(634)

<220>
<221>  sig_peptide
<222>  (41)..(94)

<220>
<221>  mat_peptide
<222>  (95)..(634)

<400>  1
actcgccgca gcctgcgcgc cttctccagt ccgcggtgcc atg gcc ccc gcc cgt        55
                                            Met Ala Pro Ala Arg
                                                        -15

ctg ttc gcg ctg ctg ctg ttc ttc gta ggc gga gtc gcc gag tcg atc       103
Leu Phe Ala Leu Leu Leu Phe Phe Val Gly Gly Val Ala Glu Ser Ile
            -10                 -5                  -1  1

cga gag act gag gtc atc gac ccc cag gac ctc cta gaa ggc cga tac       151
Arg Glu Thr Glu Val Ile Asp Pro Gln Asp Leu Leu Glu Gly Arg Tyr
    5                   10                  15

ttc tcc gga gcc cta cca gac gat gag gat gta gtg ggg ccc ggg cag       199
Phe Ser Gly Ala Leu Pro Asp Asp Glu Asp Val Val Gly Pro Gly Gln
20                  25                  30                  35

gaa tct gat gac ttt gag ctg tct ggc tct gga gat ctg gat gac ttg       247
Glu Ser Asp Asp Phe Glu Leu Ser Gly Ser Gly Asp Leu Asp Asp Leu
                40                  45                  50

gaa gac tcc atg atc ggc cct gaa gtt gtc cat ccc ttg gtg cct cta       295
Glu Asp Ser Met Ile Gly Pro Glu Val Val His Pro Leu Val Pro Leu
            55                  60                  65

gat aac cat atc cct gag agg gca ggg tct ggg agc caa gtc ccc acc       343
Asp Asn His Ile Pro Glu Arg Ala Gly Ser Gly Ser Gln Val Pro Thr
        70                  75                  80

gaa ccc aag aaa cta gag gag aat gag gtt atc ccc aag aga atc tca     391
Glu Pro Lys Lys Leu Glu Glu Asn Glu Val Ile Pro Lys Arg Ile Ser
    85                  90                  95

ccc gtt gaa gag agt gag gat gtg tcc aac aag gtg tca atg tcc agc     439
Pro Val Glu Glu Ser Glu Asp Val Ser Asn Lys Val Ser Met Ser Ser
100                 105                 110                 115

act gtg cag ggc agc aac atc ttt gag aga acg gag gtc ctg gca gct     487
Thr Val Gln Gly Ser Asn Ile Phe Glu Arg Thr Glu Val Leu Ala Ala
                120                 125                 130

ctg att gtg ggt ggc atc gtg ggc atc ctc ttt gcc gtc ttc ctg atc     535
Leu Ile Val Gly Gly Ile Val Gly Ile Leu Phe Ala Val Phe Leu Ile
            135                 140                 145

cta ctg ctc atg tac cgt atg aag aag aag gat gaa ggc agc tat gac     583
Leu Leu Leu Met Tyr Arg Met Lys Lys Lys Asp Glu Gly Ser Tyr Asp
        150                 155                 160

ctg ggc aag aaa ccc atc tac aag aaa gcc ccc acc aat gag ttc tac     631
Leu Gly Lys Lys Pro Ile Tyr Lys Lys Ala Pro Thr Asn Glu Phe Tyr
    165                 170                 175

gcg tgaagcttgc ttgtgggcac tggcttggac tttagcgggg agggaagcca     684
Ala
180

ggggattttg aagggtggac attagggtag ggtgaggtca acctaatact gacttgtcag     744

tatctccagc tctgattacc tttgaagtgt tcagaagaga cattgtcttc tactgttctg     804

ccaggttctt cttgagcttt gggcctcagt tgccctggca gaaaaatgga ttcaacttgg     864

cctttctgaa ggcaagactg ggattggatc acttcttaaa cttccagtta agaatctagg     924

tccgccctca agcccatact gaccatgcct catccagagc tcctctgaag ccagggggct     984

aacggatgtt gtgtggagtc ctggctggag gtcctccccc agtggccttc ctcccttcct     1044

ttcacagccg gtctctctgc caggaaatgg gggaaggaac tagaaccacc tgcaccttga     1104

gatgtttctg taaatgggta cttgtgatca cactacggga atctctgtgg tatatacctg     1164

gggccattct aggctctttc aagtgacttt tggaaatcaa ccttttttat ttggggggga     1224

ggatggggaa aagagctgag agtttatgct gaaatggatt tatagaatat ttgtaaatct     1284

atttttagtg tttgttcgtt tttttaactg ttcattcctt tgtgcagagt gtatatctct     1344

gcctgggcaa gagtgtggag gtgccgaggt gtcttcattc tctcgcacat ttccacagca     1404

cctgctaagt ttgtatttaa tggtttttgt ttttgttttt gtttgtttct tgaaaatgag     1464

agaagagccg gagagatgat ttttattaat tttttttttt tttttttttt tttactattt     1524

atagctttag atagggcctc ccttcccctc ttctttcttt gttctctttc attaaacccc     1584

ttccccagtt ttttttttat actttaaacc ccgctcctca tggccttggc cctttctgaa     1644

gctgcttcct cttataaaat agcttttgcc gaaacatagt ttttttttag cagatcccaa     1704

aatataatga aggggatggt gggatatttg tgtctgtgtt cttataatat attattattc     1764

ttccttggtt ctagaaaaat agataaatat atttttttca ggaaatagtg tggtgtttcc 1824

agtttgatgt tgctgggtgg ttgagtgagt gaattttcat gtggctgggt gggtttttgc 1884

ctttttctct tgccctgttc ctggtgcctt ctgatggggc tggaatagtt gaggtggatg 1944

gttctaccct ttctgccttc tgtttgggac ccagctggtg ttctttggtt tgctttcttc 2004

aggctctagg gctgtgctat ccaatacagt aaccacatgc ggctgtttaa agttaagcca 2064

attaaaatca cataagatta aaaattcctt cctcagttgc actaaccacg tttctagagg 2124

cgtcactgta tgtagttcat ggctactgta ctgacagcga gagcatgtcc atctgttgga 2184

cagcactatt ctagagaact aaactggctt aacgagtcac agcctcagct gtgctgggac 2244

gacccttgtc tccctgggta gggggggggg aatgggggag ggctgatgag gccccagctg 2304

gggcctgttg tctgggaccc tccctctcct gagaggggag gcctggtggc ttagcctggg 2364

caggtcgtgt ctcctcctga ccccagtggc tgcggtgagg ggaaccaccc tcccttgctg 2424

caccagtggc cattagctcc cgtcaccact gcaacccagg gtcccagctg gctgggtcct 2484

cttctgcccc cagtgccctt ccccttgggc tgtgttggag tgagcacctc ctctgtaggc 2544

acctctcaca ctgttgtctg ttactgattt tttttgataa aaagataata aaacctggta 2604

ctttctaaaa a 2615

<210> 2
<211> 198
<212> PRT
<213> Homo sapiens

<400> 2

Met Ala Pro Ala Arg Leu Phe Ala Leu Leu Leu Phe Phe Val Gly Gly
-15 -10 -5

Val Ala Glu Ser Ile Arg Glu Thr Glu Val Ile Asp Pro Gln Asp Leu
-1 1 5 10

Leu Glu Gly Arg Tyr Phe Ser Gly Ala Leu Pro Asp Asp Glu Asp Val
15 20 25 30

Val Gly Pro Gly Gln Glu Ser Asp Asp Phe Glu Leu Ser Gly Ser Gly
35 40 45

Asp Leu Asp Asp Leu Glu Asp Ser Met Ile Gly Pro Glu Val Val His
50 55 60

Pro Leu Val Pro Leu Asp Asn His Ile Pro Glu Arg Ala Gly Ser Gly
65 70 75

Ser Gln Val Pro Thr Glu Pro Lys Lys Leu Glu Glu Asn Glu Val Ile
    80                  85                  90

Pro Lys Arg Ile Ser Pro Val Glu Glu Ser Glu Asp Val Ser Asn Lys
95                  100                 105                 110

Val Ser Met Ser Ser Thr Val Gln Gly Ser Asn Ile Phe Glu Arg Thr
                115                 120                 125

Glu Val Leu Ala Ala Leu Ile Val Gly Gly Ile Val Gly Ile Leu Phe
            130                 135                 140

Ala Val Phe Leu Ile Leu Leu Leu Met Tyr Arg Met Lys Lys Lys Asp
        145                 150                 155

Glu Gly Ser Tyr Asp Leu Gly Lys Lys Pro Ile Tyr Lys Lys Ala Pro
    160                 165                 170

Thr Asn Glu Phe Tyr Ala
175                 180

<210>  3
<211>  21
<212>  DNA
<213>  Artificial Sequence

<220>
<223>  Sense strand of siRNA #6

<400>  3
gguccuggca gcucugauut t                                                21

<210>  4
<211>  21
<212>  DNA
<213>  Artificial Sequence

<220>
<223>  Antisense strand of siRNA #6

<400>  4
aaucagagcu gccaggacct t                                                21

<210>  5
<211>  21
<212>  DNA
<213>  Artificial Sequence

<220>
<223>  Sense strand of siRNA #7

<400>  5
aggaugaagg cagcuaugat t                                                21

<210>  6
<211>  21
<212>  DNA
<213>  Artificial Sequence

<220>
<223>  Antisense strand of siRNA #7

<400>  6
ucauagcugc cuucauccut t                                                21


<210>  7
<211>  21
<212>  DNA
<213>  Artificial Sequence

<220>
<223>  Sense strand of siRNA #10

<400>  7
gcaagaaacc caucuacaat t                                                21


<210>  8
<211>  21
<212>  DNA
<213>  Artificial Sequence

<220>
<223>  Antisense strand of siRNA #10

<400>  8
uuguagaugg guuucuugct t                                                21


<210>  9
<211>  21
<212>  DNA
<213>  Artificial Sequence

<220>
<223>  Sense strand of siRNA #1

<400>  9
cuacugcuca uguaccguat t                                                21


<210>  10
<211>  21
<212>  DNA
<213>  Artificial Sequence

<220>
<223>  Antisense strand of siRNA #1

<400>  10
uacgguacau gagcaguagg a                                                21


<210>  11
<211>  21

<212>  DNA
<213>  Artificial Sequence

<220>
<223>  Sense strand of siRNA #2

<400>  11
gcuaugaccu gggcaagaat t                                                21


<210>  12
<211>  21
<212>  DNA
<213>  Artificial Sequence

<220>
<223>  Antisense strand of siRNA #2

<400>  12
uucuugccca ggucauagct g                                                21


<210>  13
<211>  21
<212>  DNA
<213>  Artificial Sequence

<220>
<223>  Sense strand of siRNA #3

<400>  13
ccaagaaacu agaggagaat t                                                21


<210>  14
<211>  21
<212>  DNA
<213>  Artificial Sequence

<220>
<223>  Antisense strand of siRNA #3

<400>  14
uucuccucua guuucuuggg t                                                21


<210>  15
<211>  19
<212>  DNA
<213>  Artificial Sequence

<220>
<223>  Sense strand of negative control siRNA

<400>  15
auccgcgcga uaguacgua                                                   19


<210>  16
<211>  19
<212>  DNA
<213>  Artificial Sequence

<220>
<223>  Antisense strand of negative control siRNA

<400>  16
auccgcgcga uaguacgua                                                    19

## Description

### [Technical Field]

The present invention relates to Syndecan4 (SDC4) which is a cancer stem cell marker and a cancer stem cell-specific drug discovery target, a method for detecting a cancer stem cell using same as an index, and a cancer stem cell-specific anticancer drug targeting same.

### [Background Art]

In recent years, it has been clarified that only a subset of cancer cells called cancer stem cells can reconstruct cancer tissue, and cause both recurrence and metastasis. Cancer stem cells show resistance to anticancer agents and radiation therapy because their division speed is slow, and are considered to cause poor prognosis of various types of cancer. Conversely, a permanent cure can also be expected by attacking cancer stem cells. Accordingly, search for markers that identify cancer stem cells and analysis of the properties of the identified cancer stem cells have been actively conducted.

A hypometabolic cell population with extremely low proteasome activity is present in cancer cells, and the association between low-proteasome activity cells and cancer stem cells in various cancers has been reported (e.g., non-patent documents 1, 2). The present inventors introduced fluorescence-labeled ornithine decarboxylase (ODC)-degron into the pancreatic cancer cell line Panc-1 to visualize hypometabolism. Cells with reduced proteasome activity cannot degrade ODC-degron, and fluorescent protein (ZsGreen) is accumulated and visualized. The present inventors sorted fluorescent cells (dZsG+) and concentrated the cells from 0.06% to 81.18% of the total. dZsG+ cells have been confirmed to have a sphere-forming ability, have resistance to anticancer agents, highly express known cancer stem cell markers Dclk1 and CD44v9, and show asymmetric division, which are kinds of properties characteristic of cancer stem cells.

When 150 non-cancer stem cells (dZsG-) and 150 cancer stem cells (dZsG+) were subcutaneously transplanted into immunodeficient mice, non-cancer stem cells did not form a tumor in all mice, whereas cancer stem cells could form a tumor 6 weeks later. When non-cancer stem cells and cancer stem cells that highly express CD44v9 (dZsG-/CD44v9^{high} and dZsG+/CD44v9^{high}) (150 cells each) were subcutaneously transplanted to immunodeficient mice, dZsG-/CD44v9^{high} did not form a tumor in all mice, whereas dZsG+/CD44v9^{high} formed a tumor.

Tumors formed by transplanting dZsG-/CD44v9- (10⁶ cells) into immunodeficient mice (tumor group 1) and tumors formed by transplanting dZsG+/CD44v9- and dZsG+/CD44v9^{high} (tumor could be formed by transplanting 150 cells; tumor group 3 and tumor group 4, respectively) were subjected to gene expression analysis using a next-generation sequencer. As a result, the principal component analysis revealed that genetic difference existed among the three. The molecular functional analysis of such genetic difference was studied using molecular network-pathway analysis. As a result, the pathways activated in tumor group 4 as compared with tumor group 1 were found to include those involved in the proliferation or progression of tumor tissues and metastasis, differentiation or proliferation of tumor cells, whereas the pathways activated in tumor group 3 as compared with tumor group 1 were not related to tumor. Thus, it was clarified that tumor group 4 is a highly active tumor cell population.

Accordingly, the tumor of tumor group 4 was singularized to establish a cell line (super Panc-1 CSC). A population of dZsG+/CD44v9^{high} was sorted from the super Panc-1 CSC, and transplanted one by one into immunodeficient mice. As a result, tumorigenesis was found on day 45, and explosive tumor growth was seen during the next 2 weeks. Thus, the present inventors have succeeded in obtaining cancer stem cells having tumorigenicity from a single cell.

CD44v9, a splicing variant of CD44, is known as a cancer stem cell marker in various cancers such as colorectal cancer, pancreatic cancer, breast cancer, gastric cancer, and prostate cancer. It has been reported that CD44v9 is not only a marker but also involved in the growth of cancer tissue and the acquisition of treatment resistance.

### [Document List]

### [Non-patent documents]

non-patent document 1: Vlashi, E. et al., J. Natl. Cancer Inst., 101: 350-359 (2009)
non-patent document 2: Adikrisna, R. et al., Gastroenterology, 143: 234-245 (2012)

### [Summary of Invention]

### [Technical Problem]

The present invention aims to provide a novel marker molecule that can be not only a marker molecule capable of specifically detecting cancer stem cells but also a therapeutic target that kills cancer stem cells, and provide a novel detection method of cancer stem cells, and a therapeutic drug targeting cancer stem cells and the like.

### [Solution to Problem]

The present inventors have conducted intensive studies in an attempt to achieve the aforementioned goal and found that Syndecan-4 (SDC4), which is a heparan sulfate proteoglycan and belongs to the Syndecan family, is more highly expressed in cancer stem cells Panc-1 dZsG+/CD44v9^{high} than in non-cancer stem cells Panc-1 dZsG-/CD44v9- of pancreatic cancer. The cultured cells of a tumor produced by transplantation of 10⁶ non-cancer stem cells, and the cultured cells from a tumor produced by transplantation of 150 cancer stem cells were subjected to Western blot analysis. As a result, it was clarified that both SDC4 and CD44v9 were more highly expressed in the latter than in the former, and the both were in parallel at the protein level. Colocalization of both was also confirmed by cell immunostaining.

dZsG+/CD44v9^{high} and dZsG+/SDC4^{high} cells were respectively sorted from pancreatic cancer stem cell super Panc-1 CSC established by the present inventors, transplanted into immunodeficient mice one by one, and the tumorigenicity was compared. As a result, it was found that the latter has a higher tumorigenicity rate and SDC4 is superior to CD44v9 as a cancer stem cell marker. Furthermore, when dZsG+/SDC4^{high} cells were transplanted together with FGF2 which is a SDC4 ligand, the tumorigenicity rate increased to about two-fold, which suggests that SDC4 signaling contributes to the property of cancer stem cells. Tissue fluorescence staining of tumors originating from a single dZsG+/CD44v9^{high} and dZsG+/SDC4^{high} cell revealed almost no CD44v9-positive cell, whereas SDC4-positive cells were highly frequently observed.

CD44v9^{high} and SDC4^{high} cells were respectively sorted from super Panc-1 CSC, and intraperitoneally administered to immunodeficient mice, and the latter showed remarkable tumorigenesis and body weight loss. Furthermore, CD44v9^{high} and SDC4^{high} cells were respectively sorted from non-genetically engineered pancreatic cancer cell lines Panc-1 and Bxpc3, and subcutaneously transplanted to immunodeficient mice. As a result, the latter showed higher tumorigenicity rate than the former in any cell lines. Moreover, CD44v9^{high} and SDC4^{high} cells were respectively sorted from Bxpc3, and intraperitoneally administered to immunodeficient mice to find remarkable tumorigenesis and body weight loss. That is, it was clarified that cancer stem cells can be efficiently sorted from a cancer cell population by using high expression of SDC4 alone as an index, even without sorting a hypometabolic cell population.

As a result of tissue fluorescence staining for CD44v9 and SDC4 in the excised cancer tissue from colorectal cancer patients, expression of CD44v9 was not observed in some cases, whereas SDC4 was highly expressed in all samples. Fluorescence immunostaining of tumor samples of a PDX model of excised cancer tissue from a colorectal cancer patient and a cell line derived from the model showed high expression of SDC4, but no expression of CD44v9. Furthermore, SDC4 high-expressing cells and low-expressing cells were respectively sorted from the aforementioned cell line derived from the PDX model, and intraperitoneally administered to immunodeficient mice. As a result, remarkable tumorigenesis was found in the former. Similarly, in the esophageal cancer cell line TE4, remarkable tumorigenesis was also observed in SDC4 high-expressing cells. From these results, it was clarified that SDC4 can be an effective cancer stem cell marker in various cancer types in human clinical practice.

Next, to investigate whether SDC4 can be a therapeutic target for cancer stem cells, 3 siRNA species against SDC4 originally designed by the present inventors and commercially-available 3 siRNA species against SDC4 were respectively introduced into super Panc-1 CSC and a cell line derived from the PDX model of excised cancer tissue from colorectal cancer patients. As a result, two kinds of siRNA showed a marked antitumor effect. The antitumor effect of these siRNAs was further enhanced when used in combination with chemotherapeutic agents and mTOR inhibitors. When used in combination with a Wnt inhibitor, one siRNA species showed a marked antitumor effect. These SDC4 siRNAs did not show toxicity to normal cells and showed highly selective toxicity to cancer stem cells. In addition, SDC4 siRNA exhibited an antitumor effect not only in vitro but also in vivo.

As a result of Western blot analysis, it was considered that the continuous reduction of SDC4 protein expression was necessary for exhibition of the antitumor effect of siRNA against SDC4. To investigate the mechanism of antitumor effect of siRNA against SDC4 on cancer stem cells, RNA was extracted from pancreatic cancer stem cells into which siRNA affording an antitumor effect and control siRNA were respectively introduced, and IPA analysis was performed on a group of genes whose expression varied markedly. As a result, suppression of upstream regulators such as mTOR, PPARγ1A, Jnk, ERK1/2, ERK, Creb and the like was predicted. In addition, treatment of pancreatic cancer stem cells with a Wnt inhibitor resulted in increased expression of SDC4, suggesting the involvement of SDC4 in Wnt signaling.

Solid tumors formed from a single cell obtained by respectively sorting dZsG+/CD44v9^{high} and dZsG+/SDC4^{high} cells from super Panc-1 CSC was immunostained for HIF1α and SDC4. As a result, many SDC4-positive cells were confirmed in HIF1α nuclear staining positive (hypoxic region), which was consistent with the finding that cancer stem cells are localized in the hypoxic region.

Similar to siRNA, an antibody against SDC4 also showed a remarkable antitumor effect on various cancer cells and cancer stem cells both in vitro and in vivo. Anti-SDC4 antibody also did not show toxicity to normal cells, and highly selective toxicity to cancer stem cells was observed.

According to the Cancer Cell Line Encyclopedia (CCLE) database, the expression level of Sdc4 mRNA in mesothelioma cells was high and 3rd place overall. Thus, the present inventors investigated the effects of SDC4 siRNA, anti-SDC4 antibody on the mesothelioma cell line. As a result, both SDC4 siRNA and anti-SDC4 antibody showed a remarkable antitumor effect on the mesothelioma cell line. A particularly strong antitumor effect was observed for mesothelioma cancer stem cells sorted using CD24 positivity as an index, which is a cancer stem cell marker for mesothelioma.

Based on these findings, the present inventors have found that SDC4 is a cancer stem cell marker superior to CD44v9, which is a known cancer stem cell marker, and that it becomes a drug discovery target of a therapeutic drug for cancer stem cells, and succeeded in developing a medicament that efficiently eliminates cancer stem cells by suppressing the expression of SDC, which resulted in the completion of the present invention.

That is, the present invention provides the following.
[1] A cancer stem cell elimination agent comprising a substance that suppresses an expression or function of SDC4.
[2] The agent of [1], wherein the substance that suppresses the expression of SDC4 is
   (a) a nucleic acid having RNAi activity against a transcription product of SDC4 gene, or a precursor thereof,
   (b) an antisense nucleic acid against a transcription product of SDC4 gene, or
   (c) a ribozyme nucleic acid against a transcription product of SDC4 gene.
[3] The agent of [2], wherein the substance that suppresses the expression of SDC4 is a nucleic acid comprising a nucleotide sequence complementary to a sequence consisting of at least 15 consecutive nucleotides in the region indicated by nucleotide numbers 41 - 637 in the nucleotide sequence shown in SEQ ID NO: 1, or a precursor thereof.
[4] The agent of [2], wherein the substance that suppresses the expression of SDC4 is a nucleic acid comprising a nucleotide sequence complementary to a sequence consisting of at least 15 consecutive nucleotides in the region indicated by nucleotide numbers 475 - 606 in the nucleotide sequence shown in SEQ ID NO: 1, or a precursor thereof.
[5] The agent of [3] or [4], wherein the agent is siRNA.
[6] The agent of [1], wherein the substance that suppresses the function of SDC4 is
   (a) an antibody against SDC4, or
   (b) an antagonist against SDC4.
[7] The agent of any of [1] to [6], wherein the agent is in combination with other antitumor drug.
[8] The agent of [7], wherein said other antitumor drug is one or more kinds selected from a chemotherapy drug, a Wnt inhibitor and an mTOR inhibitor.
[9] A method for detecting or sorting a cancer stem cell in a cancer cell population, comprising using an expression of SDC4 as an index.
[10] A reagent for detecting a cancer stem cell, comprising an antibody against SDC4.

### [Advantageous Effects of Invention]

According to the present invention, since an antitumor drug targeting a cancer stem cell can be provided, recurrence and metastasis of cancer can be suppressed and a permanent cure of cancer can be provided. According to the present invention, moreover, since a cancer stem cell marker superior to known cancer stem cell markers can be provided, detection and sorting of a cancer stem cell is facilitated.

### [Brief Description of Drawings]

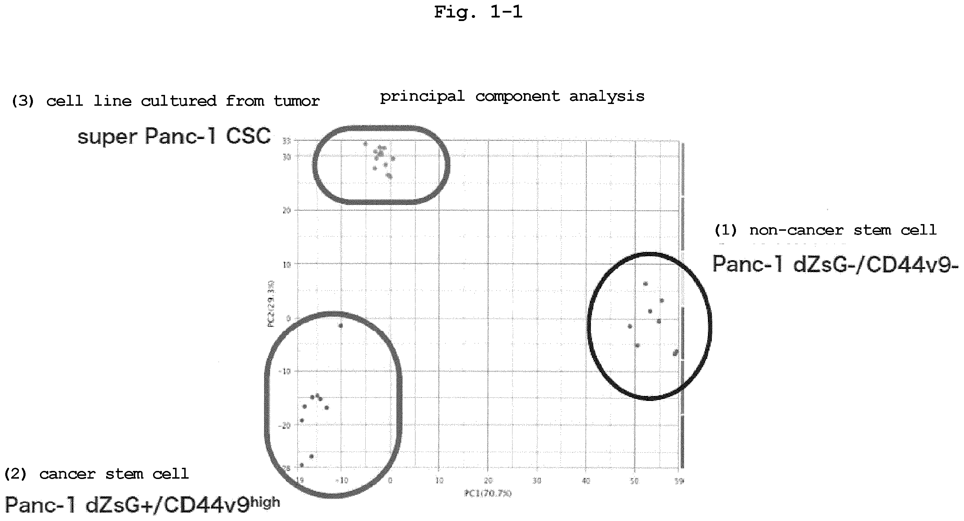

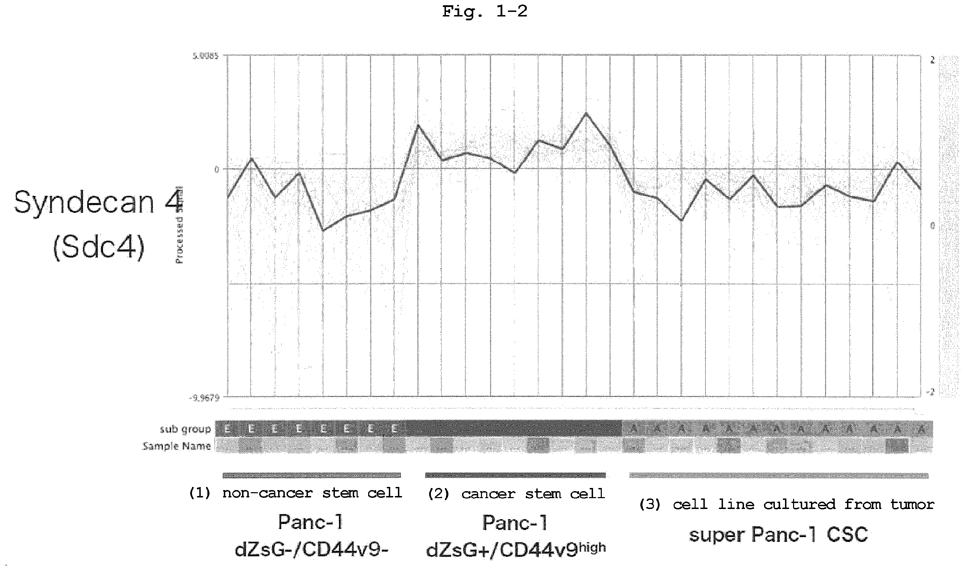

Fig. 1-1 shows the results of principal component analysis of gene expression in Panc-1 dZsG-/CD44v9-, Panc-1 dZsG+/CD44v9^{high}, and super Panc-1 CSC.
Fig. 1-2 shows comparison of the expression of SDC4 gene in Panc-1 dZsG-/CD44v9-, Panc-1 dZsG+/CD44v9^{high}, and super Panc-1 CSC.
Fig. 2-1 shows expression of SDC4 and CD44v9 in 1-1: dZsG-/CD44v9-, 1-2: dZsG-/CD44v^{high}, 3-3: dZsG+/CD44v9-, 3-4: dZsG+/CD44v9^{high}.
Fig. 2-2 shows the results of cell immunostaining showing the expression of SDC4 and CD44v9 in dZsG+/CD44v9^{high} cells of super Panc-1 CSC. red: CD44v9 green: SDC4 blue: nucleus
Fig. 3-1 shows that SDC4 is a cancer stem cell marker superior to CD44v9, using the tumorigenicity at a single cell level as an index.
Fig. 3-2 shows the results of the tissue immunofluorescence staining of tumor formed from a single cell. CD44v9-positive cells were not found and many Sdc4-positive cells were found, thus indicating that SDC4 is a cancer stem cell marker superior to CD44v9. HE: Hematoxylin-Eosin staining
Fig. 4-1 shows that SDC4 is a cancer stem cell marker superior to CD44v9, using the tumorigenicity by intraperitoneal administration as an index (super Panc-1 CSC).
Fig. 4-2 shows that SDC4 is a cancer stem cell marker superior to CD44v9, using the tumorigenicity by intraperitoneal administration as an index (Bxpc3).
Fig. 5-1 shows that SDC4 is a cancer stem cell marker superior to CD44v9, using the tumorigenicity by subcutaneous injection as an index (Panc-1).
Fig. 5-2 shows that SDC4 is a cancer stem cell marker superior to CD44v9, using the tumorigenicity by subcutaneous injection as an index (Bxpc3).
Fig. 6-1 shows the results of the tissue immunofluorescence staining for CD44v9 and SDC4 in the excised cancer tissue of colorectal cancer patients (sample No. 2). In the high malignancy region, CD44v9-positive cells were not found and many Sdc4-positive cells were found. green: CD44v9 red: SDC4 blue: nucleus
Fig. 6-2 shows the results of the tissue immunofluorescence staining for CD44v9 and SDC4 in the excised cancer tissue of colorectal cancer patients (sample No. 4). CD44v9-positive cells were not found and many Sdc4-positive cells were found. green: CD44v9 red: SDC4 blue: nucleus
Fig. 6-3 shows the results of the tissue immunofluorescence staining for CD44v9 and SDC4 in the excised cancer tissue of colorectal cancer patients (sample No. 6). green: CD44v9 red: SDC4 blue: nucleus
Fig. 6-4 shows the results of the tissue immunofluorescence staining for CD44v9 and SDC4 in the excised cancer tissue of colorectal cancer patients (sample No. 8). green: CD44v9 red: SDC4 blue: nucleus
Fig. 7-1 shows the results of the tissue immunofluorescence staining for CD44v9 and SDC4 in a PDX model of the excised cancer tissue of colorectal cancer patients (sample No. 2). CD44v9-positive cells were not found and many Sdc4-positive cells were found. green: CD44v9 red: SDC4 blue: nucleus
Fig. 7-2 shows the results of the tissue immunofluorescence staining for CD44v9 and SDC4 in a PDX model-derived cell line (PDX No. 2) of the excised cancer tissue of colorectal cancer patients. green: CD44v9 red: SDC4 blue: nucleus
Fig. 8-1 shows that SDC4 is a superior cancer stem cell marker of colorectal cancer using the tumorigenicity by intraperitoneal administration as an index (PDX No. 2).
Fig. 8-2 shows that SDC4 is a superior cancer stem cell marker of colorectal cancer using the tumorigenicity by intraperitoneal administration as an index (PDX No. 2).
Fig. 8-3 shows that SDC4 is a superior cancer stem cell marker of esophageal cancer using the tumorigenicity by intraperitoneal administration as an index (TE4).
Fig. 9-1 shows an antitumor cell effect by siRNA targeting SDC4.
Fig. 9-2 shows an antitumor cell effect by combined use of siRNA targeting SDC4 and a chemotherapeutic agent.
Fig. 9-3 shows an antitumor cell effect by combined use of siRNA targeting SDC4 and Wnt inhibitor FH535.
Fig. 9-4 shows an antitumor cell effect by combined use of siRNA targeting SDC4 and Wnt inhibitor PNU.
Fig. 9-5 shows an antitumor cell effect by combined use of siRNA targeting SDC4 and mTOR inhibitor.
Fig. 9-6 shows the results of a toxicity test of siRNA targeting SDC4 on normal cells and cancer stem cells.
Fig. 9-7 shows the expression of SDC4 in normal cells and cancer stem cells.
Fig. 9-8 shows an antitumor effect of siRNA targeting SDC4 on mouse subcutaneous solid tumor model.
Fig. 10-1 shows the relationship between the antitumor cell effect by siRNA targeting SDC4 and SDC4 expression level.
Fig. 10-2 shows the analysis results of the mechanism of an antitumor cell effect of siRNA targeting SDC4 by the upstream analysis of IPA.
Fig. 10-3 shows that SDC4 expression is induced by a Wnt inhibitor in super Panc-1 CSC.
Fig. 11 shows that SDC4 is expressed in a hypoxic region in a tumor formed from a single cancer stem cell. (a) - (c): solid tumors derived from dZsG+/CD44v9^{high} of super Panc-1 CSC. (d) - (f) : solid tumors derived from dZsG+/SDC4^{high} of super Panc-1 CSC.
Fig. 12-1 shows an antitumor effect of Sdc4 antibody on colorectal cancer.
Fig. 12-2 shows an antitumor effect of Sdc4 antibody on colorectal cancer.
Fig. 12-3 shows an antitumor effect of Sdc4 antibody on pancreatic cancer.
Fig. 12-4 shows an antitumor effect of Sdc4 antibody on pancreatic cancer.
Fig. 12-5 shows the results of a toxicity test of Sdc4 antibody to normal cells.
Fig. 12-6 shows the results of a toxicity test of Sdc4 antibody to normal cells.
Fig. 12-7 shows an antitumor effect of SDC4 antibody in a mouse subcutaneous solid tumor model.
Fig. 13-1 shows an antitumor cell effect of siRNA targeting SDC4 on the mesothelioma cell line.
Fig. 13-2 shows an antitumor cell effect of SDC4 antibody on the mesothelioma cell line.
Fig. 13-3 shows an antitumor cell effect of SDC4 antibody on the mesothelioma cancer stem cell.

### [Description of Embodiments]

The present invention provides a cancer stem cell elimination agent containing a substance that suppresses the expression or function of Syndecan-4 (SDC4) (hereinafter to be also referred to as "the CSC elimination agent of the present invention").

In the present specification, the "cancer stem cell (CSC)" means a cancer cell that has self-renewal ability and pluripotency, has high tumorigenicity and metastatic ability, is resistant to chemotherapeutic agents and radiation therapy, and is characterized by asymmetric division from stem cells to stem cells and non-stem cells.

In the present specification, the type of cancer of the cancer stem cell is not particularly limited and any cancer can be mentioned. For example, it may be a cancer derived from epithelial cells or may be non-epithelial sarcoma or hematologic cancer. More specifically, for example, it includes, but is not limited to, gastrointestinal cancer (e.g., esophageal cancer, stomach cancer, duodenum cancer, colorectal cancer (colon cancer, rectal cancer), liver cancer (hepatocellular cancer, cholangiocellular cancer), gall bladder cancer, bile duct cancer, pancreatic cancer, anal cancer), urinary organ cancer (e.g., kidney cancer, ureteral cancer, bladder cancer, prostate cancer, penile cancer, testis (orchis) cancer), thorax cancer (e.g., breast cancer, lung cancer (non-small cell lung cancer, small cell lung cancer)), cancer of reproductive organ(e.g., uterine cancer (uterus cervix cancer, cancer of the uterine body), ovarian cancer, vulvar cancer, vaginal cancer), head and neck cancer (e.g., maxilla cancer, pharyngeal cancer, laryngeal cancer, tongue cancer, thyroid cancer), skin cancer (e.g., basal cell carcinoma, squamous cell carcinoma), and mesothelial cell cancer (mesothelioma). Preferably, colorectal cancer, pancreatic cancer, esophageal cancer, mesothelioma, breast cancer, stomach cancer, prostate cancer and the like, more preferably colorectal cancer, pancreatic cancer, mesothelioma and the like, can be mentioned.

SDC4, which is the target molecule of the CSC elimination agent of the present invention, is a single-pass transmembrane type protein and is a heparan sulfate proteoglycan belonging to the Syndecan family. In human, it has an amino acid sequence consisting of 198 amino acids shown in SEQ ID NO: 2, of which the 1- to 18-positions are signal peptides, the 19- to 145-positions indicate an extracellular region, the 146- to 170-positions indicate a transmembrane region, and the 171- to 198-positions indicate an intracellular region.

In the present specification, "SDC4" is a protein having an amino acid sequence the same or substantially the same as the amino acid sequence shown in SEQ ID NO: 2. In the present specification, the protein and peptide are described according to the common practice of peptide designation, wherein the left end indicates the N-terminal (amino terminal) and the right end indicates the C-terminal (carboxyl terminal).

The "amino acid sequence substantially the same as the amino acid sequence shown in SEQ ID NO: 2" means
(a) the amino acid sequence of an ortholog of human SDC4 consisting of the amino acid sequence shown in SEQ ID NO: 2 in other warm-blooded animal (e.g., guinea pig, rat, mouse, chicken, rabbit, dog, swine, sheep, bovine, monkey and the like); or
(b) the amino acid sequence of a natural allelic variant or polymorphic form of the human SDC4 consisting of the amino acid sequence shown in SEQ ID NO: 2 or ortholog of the above-mentioned (a).

Preferably, SDC4 is a human SDC4 consisting of the amino acid sequence shown in SEQ ID NO: 2 or a natural allelic variant or polymorphic form thereof. Examples of the gene polymorphism include, but are not limited to, SNP in which the 12-position Phe(TTC) is substituted by Leu(CTC) which is registered as rs2228384 in dbSNP.

In the present invention, the "substance that suppresses the expression of SDC4" may act at any stage, such as the transcription level, the level of post-transcriptional regulation, the level of translation into proteins, the level of post-translational modification, and the like of SDC4 gene. Therefore, examples of the substance that suppresses the expression of SDC4 include substances that inhibit the transcription of SDC4 gene (e.g., antigene), substances that inhibit the processing of initial transcription products to mRNA, substances that inhibit the transport of mRNA to the cytoplasm, substances that inhibit the translation of mRNA into protein (e.g., antisense nucleic acid, miRNA) or degrade mRNA (e.g., siRNA, ribozyme, miRNA), substances that inhibit post-translational modification of initial translation products and the like. While any substance that acts at any stage can be used, a substance that complementarily binds to the mRNA and inhibits translation into a protein or degrades the mRNA is preferable.

As the substance that specifically inhibits translation of mRNA of SDC4 gene into a protein (or degrades mRNA), preferably, a nucleic acid containing (a part of) a nucleotide sequence complementary to the nucleotide sequence of the mRNA can be mentioned.

The nucleotide sequence complementary to the nucleotide sequence of the mRNA of SDC4 gene means a nucleotide sequence having complementarity of the level that permits binding to the target sequence of the mRNA and inhibiting its translation (or cleaving the target sequence) under physiological conditions. Specifically, for example, it is a nucleotide sequence having a homology of 90% or more, preferably 95% or more, more preferably 97% or more, particularly preferably 98% or more, to the nucleotide sequence completely complementary to the nucleotide sequence of the mRNA (that is, nucleotide sequence of the complementary strand of mRNA) for the overlapping region. The "homology of the nucleotide sequence" in the present specification can be calculated using the homology calculation algorithm NCBI BLAST (National Center for Biotechnology Information Basic Local Alignment Search Tool) under the following conditions (expectation=10; gap allowed; filtering=ON; match score=1; mismatch score=-3).

More specifically, the nucleotide sequence complementary to the nucleotide sequence of the mRNA of SDC4 gene means a nucleotide sequence that hybridizes with the nucleotide sequence shown in SEQ ID NO: 1 under stringent conditions. As the "stringent conditions" here, for example, the conditions described in Current Protocols in Molecular Biology, John Wiley & Sons, 6.3.1-6.3.6, 1999, for example, conditions of a hybridization reaction in 6×SSC (sodium chloride/sodium citrate) at 45°C, followed by washing in 0.2×SSC/0.1% SDS/50-65°C once or more and the like can be mentioned. Those skilled in the art are able to appropriately select hybridization conditions that afford equivalent stringency.

Preferable examples of the mRNA of SDC4 gene include mRNAs of human SDC4A containing the nucleotide sequence shown in SEQ ID NO: 1 (RefSeq Accession No. NM_002999), orthologs thereof in other warm-blooded animal, natural allelic variants or polymorphic form thereof, and the like.

The "part of the nucleotide sequence complementary to the nucleotide sequence of the mRNA of SDC4 gene" is not particularly limited in terms of the length and position as long as it can specifically bind to mRNA of SDC4 gene and can inhibit translation of the mRNA into a protein (or degrade the mRNA). From the aspect of sequence specificity, it contains at least 10 bases or more, preferably 15 bases or more, and more preferably 19 bases or more, that are complementary to the target sequence.

Specifically, as a nucleic acid containing a part of the nucleotide sequence complementary to the nucleotide sequence of the mRNA of SDC4 gene, any of the following (a) - (c) is preferably recited:
(a) nucleic acid having RNAi activity against mRNA of SDC4 gene, or a precursor thereof
(b) antisense nucleic acid against mRNA of SDC4 gene
(c) ribozyme nucleic acid against mRNA of SDC4 gene.

### (a) Nucleic acid having RNAi activity against mRNA of SDC4 gene, or a precursor thereof

In the present specification, double-stranded RNA consisting of oligoRNA complementary to the mRNA of SDC4 gene and complementary strand thereof, i.e., siRNA, is defined as being encompassed in the nucleic acids containing a nucleotide sequence complementary to the nucleotide sequence of the mRNA of SDC4 gene or a part of the nucleotide sequence.

siRNA can be designed based on the cDNA sequence information of the target gene and according to the rules proposed by, for example, Elbashir et al. (Genes Dev., 15, 188-200 (2001)). Examples of the target sequence of siRNA include, but are not limited to, AA+ (N) ₁₉, AA+ (N) ₂₁, NA+ (N) ₂₁ (N is any base) and the like. The position of the target sequence is not particularly limited. For the selected target sequence candidate group, the presence of homology in the continuous sequence of 16-17 bases in non-target mRNA is examined using homology search software such as BLAST (http://www.ncbi.nlm.nih.gov/BLAST/) and the like, and the specificity of the selected target sequence is confirmed. For example, when AA+ (N) ₁₉, AA+ (N) ₂₁ or NA+ (N) ₂₁ (N is any base) is the target sequence, for the target sequence with confirmed specificity, a double-stranded RNA consisting of a sense strand having 3'-terminal overhang of TT or UU at 19-21 bases after AA (or NA), and an antisense strand having a sequence complementary to the 19-21 bases and 3'-terminal overhang of TT or UU may be designed as siRNA. Also, a short hairpin RNA (shRNA), which is a precursor of siRNA, can be designed by appropriately selecting any linker sequence (for example, about 5-25 bases) capable of forming a loop structure, and linking the above-mentioned sense strand and the antisense strand via the linker sequence.

The sequence of siRNA and/or shRNA can be searched for by using a searching software provided free of charge on various web sites. Examples of such site include, but are not limited to, siDESIGN Center provided by Dharmacon (http://dharmacon.horizondiscovery.com/jp/design-center/?rdr=true&LangType=1041&pageid=17179928204), siRNA Target Finder provided by GenScript (https://www.genscript.com/tools/sirna-target-finder) and the like. Whether siRNA or shRNA synthesized based on the hit siRNA sequence information can actually suppress SDC4 expression in cancer stem cells to a therapeutically effective extent can be verified, for example, as shown in (4-1) of Example 2 described later, by measuring the expression level of SDC4 protein in cancer stem cells 3 days after the introduction of the nucleic acid. When the SDC4 protein expression-suppressing effect diminishes within 3 days after introduction even though RNAi activity is inherently present, the SDC4 expression-suppressing effect of can be sustained by variously modifying the constituent nucleotides of siRNA or shRNA to improve in vivo stability thereof as described later, whereby the desired therapeutic effect can be achieved.

In a preferred embodiment, the siRNA and shRNA in the present invention contain a nucleotide sequence complementary to a sequence consisting of at least 15 consecutive nucleotides in the region indicated by nucleotide numbers 41 - 637 in the nucleotide sequence shown in SEQ ID NO: 1. In a particularly preferred embodiment, the siRNA and shRNA in the present invention contain a nucleotide sequence complementary to a sequence consisting of at least 15 consecutive nucleotides in the region indicated by nucleotide numbers 475 - 606, more preferably, 564 - 594 in the nucleotide sequence shown in SEQ ID NO: 1.

In the present specification, the microRNA targeting mRNA of SDC4 gene (miRNA) is also defined to be encompassed in the nucleic acid containing (a part of) a nucleotide sequence complementary to the nucleotide sequence of the mRNA of SDC4 gene. The miRNA is involved in the post-transcriptional regulation of gene expression by complementarily binding to the target mRNA and suppressing translation of the mRNA, or by degrading the mRNA.

In miRNA, a primary transcription product, primary-microRNA (pri-miRNA), is transcribed from the gene encoding the miRNA, then processed by Drosha into a precursor-microRNA (pre-miRNA) of about 70 bases in length with a characteristic hairpin structure, transported from the nucleus to the cytoplasm, further processed into mature miRNA by Dicer-mediated processing, and taken up by RISC to act on the target mRNA. Therefore, pre-miRNA or pri-miRNA, preferably pre-miRNA, can also be used as a precursor of miRNA.

miRNA can be searched for by using a target prediction software provided free of charge on various web sites. Examples of such site include, but are not limited to, TargetScan published by Whitehead Institute, USA (http://www.targetscan.org/vert_72/), DIANA-micro-T-CDS published by Alexander Fleming Biomedical Sciences Research Centre, Greece (http://diana.imis.athena-innovation.gr/DianaTools/index.php?r=microT_CDS/index) and the like. Alternatively, miRNA that targets SDC4 mRNA can also be searched for by using TarBase which is a database relating to miRNA that has been experimentally proven to act on target mRNA, and published by the Pasteur Institute, University of Chezalley, and the like (http://carolina.imis.athena-innovation.gr/diana_tools/web/index.php?r=tarbasev8/index). For example, miRNAs for SDC4 mRNA hit on the database and having a high score by the above-mentioned target prediction software include hsa-miR-1277-5p, hsa-miR-140-3p, hsa-miR-224-5p, hsa-miR-936 and the like. The sequence information of these miRNAs and/or pre-miRNAs can be obtained using, for example, miRBase published by the University of Manchester, UK (http://www.mirbase.org/search.shtml) .

The nucleotide molecules that constitute siRNA and/or shRNA, or miRNA and/or pre-miRNA, may be natural RNA or DNA. To improve stability (chemical and/or against enzyme) and specific activity (affinity for RNA), they may contain various chemical modifications. For example, to prevent degradation by hydrolases such as nuclease and the like, the phosphate residue (phosphate) of each nucleotide constituting the antisense nucleic acid can be replaced with a chemically modified phosphate residue such as phosphorothioate (PS), methylphosphonate, phosphorodithionate and the like. In addition, a hydroxyl group at the 2'-position of the sugar (ribose) of each nucleotide may be replaced with -OR (R=CH₃(2'-O-Me) , CH₂CH₂OCH₃(2'-O-MOE), CH₂CH₂NHC(NH)NH₂, CH₂CONHCH₃, CH₂CH₂CN, etc.). Furthermore, the base moiety (pyrimidine, purine) may be chemically modified and, for example, introduction of a methyl group or a cationic functional group into the 5-position of the pyrimidine base, substitution of the carbonyl group at the 2-position with thiocarbonyl, and the like can be mentioned.

In the conformation of the sugar part of RNA, C2'-endo (S type) and C3'-endo (N type) are dominant, and they exist as an equilibrium in single-stranded RNA but fixed to N-type when double-stranded RNA is formed. Therefore, BNA (LNA) (Imanishi, T. et al., Chem. Commun., 1653-9, 2002; Jepsen, J.S. et al., Oligonucleotides, 14, 130-46, 2004) and ENA (Morita, K. et al., Nucleosides Nucleotides Nucleic Acids, 22, 1619-21, 2003) which are RNA derivatives in which the conformation of the sugar part is fixed to N-type by cross-linking 2' oxygen and 4' carbon in order to impart strong binding ability to the target RNA may also be preferably used.

siRNA can be prepared by respectively synthesizing the sense strand and antisense strand of the target sequence on mRNA by a DNA/RNA automatic synthesizer, denaturing the strands in an appropriate annealing buffer at about 90 - about 95°C for about 1 min, and annealing same at about 30 - about 70°C for about 1 - about 8 hr. It can also be prepared by synthesizing shRNA, which is a precursor of siRNA, and cleaving same with a dicer. miRNA and pre-miRNA can be synthesized by a DNA/RNA automatic synthesizer based on the sequence information thereof.

In the present specification, a nucleic acid designed to produce siRNA or miRNA against mRNA of SDC4 gene in vivo is also defined to be encompassed in the nucleic acid containing (a part of) a nucleotide sequence complementary to the nucleotide sequence of the mRNA of SDC4 gene. Examples of such nucleic acid include expression vectors constructed to express the above-mentioned shRNA or siRNA or miRNA or pre-miRNA, and the like. shRNA can be prepared by designing an oligoRNA containing a nucleotide sequence in which a sense strand and an antisense strand of the target sequence on mRNA are linked by inserting therebetween a spacer sequence of a length (e.g., about 5 to 25 bases) that can form an appropriate loop structure, and synthesizing same by a DNA/RNA automatic synthesizer. Vectors expressing shRNA include tandem type and stem loop (hairpin) type. The former includes an expression cassette of the sense strand and an expression cassette of the antisense strand of siRNA linked in tandem, in which each strand is expressed and annealed in the cell to form double-stranded siRNA (dsRNA). On the other hand, the latter includes an expression cassette of shRNA inserted into a vector, in which the shRNA is expressed intracellularly and processed by a dicer to form dsRNA. As the promoter, polII type promoter (e.g., CMV early-immediate promoter) can also be used. PolIII promoters are generally used to achieve accurate transcription of short RNAs. Examples of the polIII type promoter include mouse and human U6-snRNA promoters, human H1-RNase P RNA promoter, human valine-tRNA promoter and the like. As the transcription termination signal, a sequence with 4 or more continuous Ts is used. Expression cassettes for miRNA and pre-miRNA can also be prepared in the same manner as shRNA.

The thus-constructed siRNA or shRNA or miRNA or pre-miRNA expression cassette is then inserted into a plasmid vector or virus vector. As such vector, viral vectors such as retrovirus, lentivirus, adenovirus, adeno-associated virus, herpes virus and Sendai virus, plasmids for the expression in animal cells and the like are used.

### (b) Antisense nucleic acid against mRNA of SDC4 gene

In the present invention, the "antisense nucleic acid against mRNA of SDC4 gene" refers to a nucleic acid containing (a part of) a nucleotide sequence complementary to the nucleotide sequence of the mRNA, and having a function of suppressing protein synthesis by binding to the target mRNA to form a specific and stable double strand.

Examples of the antisense nucleic acid include polydeoxyribonucleotide containing 2-deoxy-D-ribose, polyribonucleotide containing D-ribose, other type of polynucleotide which is N-glycoside of purine or pyrimidine base, other polymer with a non-nucleotide backbone (e.g., commercially available protein nucleic acids and synthetic sequence-specific nucleic acid polymer), other polymer containing a special bond (the polymer containing nucleotide with configuration that allows base pairing and base attachment as found in DNA and RNA), and the like. They may be double-stranded DNA, single-stranded DNA, double-stranded RNA, single-strand RNA, DNA:RNA hybrid, further, unmodified polynucleotide (or unmodified oligonucleotide), one with known modification, for example, one with a label known in the art, one with a cap, methylated one, one with one or more natural nucleotides substituted with analog, one with intramolecular nucleotide modification, for example, one with non-charged bond (e.g., methylphosphonate, phosphotriester, phosphoramidate, carbamate and the like), one with charged bond or sulfur-containing bond (e.g., phosphorothioate, phosphorodithioate and the like), for example, one with side chain groups such as protein (e.g., nuclease, nuclease inhibitor, toxin, antibody, signal peptide, poly-L-lysine and the like) and sugar (e.g., monosaccharide, etc.), one with intercalate compound (e.g., acridine, psoralen and the like), one containing chelate compound (e.g., metal, radioactive metal, boron, oxidative metal and the like), and one containing alkylating agent, one with modified bond (e.g., α anomer type nucleic acid and the like). As used herein, the "nucleoside", "nucleotide" and "nucleic acid" may include those containing purine and pyrimidine bases, as well as those having other modified heterocyclic bases. Such modification may include methylated purine and pyrimidine, acylated purine and pyrimidine, and other heterocycles. Modified nucleoside and modified nucleotide may also have modified sugar portion and, for example, one or more hydroxyl groups may be substituted by halogen, aliphatic group, and the like, or converted to functional group such as ether, amine, and the like.

As described above, the antisense nucleic acid may be DNA or RNA, or DNA/RNA chimera. When the antisense nucleic acid is DNA, RNA:DNA hybrid formed by target RNA and antisense DNA is recognized by endogenous RNase H and can cause selective degradation of the target RNA. Therefore, in the case of an antisense DNA intended to degrade RNA with RNase H, the target sequence may be not only a sequence in mRNA, but also a sequence of the intron region in the initial translation product of the SDC4 gene. The intron sequence can be determined by comparing the genomic sequence with the cDNA nucleotide sequence of the SDC4 gene by using a homology search program such as BLAST, FASTA and the like.

The target region of the antisense nucleic acid of the present invention is not particularly limited with respect to the length thereof, as long as the translation into a protein is inhibited as a result of hybridization of the antisense nucleic acid; the target region may be the entire sequence or a partial sequence of the mRNA that encodes the protein, and the length is about 10 bases for the shortest, and the entire sequence of the mRNA or initial transcription product for the longest. In terms of problems in synthetic easiness, antigenicity, intracellular translocation and the like, oligonucleotides are preferably, but not limited to, those consisting of about 10 - about 40 bases, in particular about 15 - about 30 bases. Specifically, the 5' end hairpin loop, 5' end 6-base-pair repeats, 5' end untranslated region, translation initiation codon, protein coding region, ORF translation stop codon, 3' end untranslated region, 3' end palindrome region, or 3' end hairpin loop and the like of the SDC4 gene may be unlimitatively chosen as a preferable target region of the antisense nucleic acid.

In one embodiment, as the target region of the antisense nucleic acid in the present invention, a sequence consisting of at least 15 consecutive nucleotides in the region indicated by nucleotide numbers 41-637 in the nucleotide sequence shown in SEQ ID NO: 1, particularly, a sequence consisting of at least 15 consecutive nucleotides in the region indicated by nucleotide numbers 475 - 606, more preferably 564 - 594, in the nucleotide sequence shown in SEQ ID NO: 1 can be mentioned, similar to the above-mentioned siRNA.

Furthermore, the antisense nucleic acid of the present invention may be not only one that inhibits translation to a protein by hybridizing with mRNA of SDC4 gene or an initial transcription product, but also one capable of inhibiting transcription to RNA (antigene) by binding to their genes, which are double-stranded DNAs, to form a triple strand (triplex).

The nucleotide molecules constituting the antisense nucleic acid may also be modified as in the above-mentioned siRNA and the like to improve stability, specific activity and the like.

The antisense oligonucleotide of the present invention can be prepared by determining the target sequence for the mRNA or initial transcription product on the basis of a cDNA sequence or genomic DNA sequence of SDC4 gene, and synthesizing a sequence complementary thereto using a commercially available DNA/RNA synthesizer (Applied Biosystems, Beckman, and the like). In addition, any antisense nucleic acids containing the above-mentioned various modifications can also be chemically synthesized by a method known per se.

### (c) Ribozyme nucleic acid against mRNA of SDC4 gene

Other examples of the nucleic acid containing (a part of) a nucleotide sequence complementary to the nucleotide sequence of the mRNA of SDC4 gene include a ribozyme nucleic acid capable of specifically cleaving the mRNA within the coding region. The "ribozyme" in a narrow sense refers to RNA having enzymatic activity for cleaving nucleic acid. In the present specification, therefore, it is used as a concept encompassing even DNA as long as it has a sequence specific enzymatic activity for cleaving nucleic acid. One of the most versatile ribozyme nucleic acids is self-splicing RNA found in infectious RNAs such as viroid and virusoid, and hammerhead type, hairpin type and the like are known. The hammerhead type exhibits enzyme activity with about 40 bases in length, and it is possible to specifically cleave only the target mRNA by making several bases (about 10 bases in total) at both ends flanking to the hammerhead structure portion a sequence complementary to the desired cleavage site of the mRNA. Because this type of ribozyme nucleic acid has only RNA as the substrate, it offers an additional advantage of non-attack of genomic DNA. When the mRNA of SDC4 gene forms a double-stranded structure per se, the target sequence can be made to be single-stranded by using a hybrid ribozyme prepared by joining an RNA motif derived from a viral nucleic acid that can bind specifically to RNA helicase [Proc. Natl. Acad. Sci. USA, 98(10): 5572-5577 (2001)]. Furthermore, when ribozyme is used in the form of an expression vector containing DNA encoding same, the ribozyme may be a hybrid ribozyme prepared by further joining a sequence modified from the tRNA to promote the translocation of the transcription product to cytoplasm [Nucleic Acids Res., 29(13): 2780-2788 (2001)].

The nucleic acid containing (a part of) a nucleotide sequence complementary to the nucleotide sequence of the mRNA of SDC4 gene may be provided in special forms such as liposome and microsphere, applied in gene therapy, or given in an added form. As those used in an added form, polycationic substances such as polylysine, which act to neutralize the charge of the phosphate group backbone, and hydrophobic substances such as lipids (e.g., phospholipid, cholesterol and the like) that enhance interaction with cell membranes and increase nucleic acid uptake can be mentioned. As a lipid preferable for addition, cholesterol and derivatives thereof (e.g., cholesterylchloroformate, cholic acid and the like) can be mentioned. These can be attached to the 3'-end or the 5'-end of nucleic acid via a base, sugar, or an intramolecular nucleoside bond. As other groups, a group for capping which is specifically provided at the 3'-end or the 5'-end of nucleic acid to prevent degradation by nuclease such as exonuclease, RNase and the like can be mentioned. Examples of the group for capping include, but are not limited to, hydroxyl protecting groups known in the art such as glycol (e.g., polyethylene glycol, tetraethyleneglycol and the like).

The SDC4 protein expression suppressing activity of these nucleic acids can be investigated using a transformant into which SDC4 gene has been introduced, an in vivo or in vitro SDC4 gene expression system, or an in vivo or in vitro SDC4 protein translation system.

In the present invention, the substance that suppresses the expression of SDC4 is not limited to the above-mentioned nucleic acid containing (a part of) a nucleotide sequence complementary to the nucleotide sequence of the mRNA of SDC4 gene, and may be other substance such as a low-molecular-weight compound and the like as long as it directly or indirectly inhibits the production of SDC4 protein.

In the present invention, the "substance that suppresses the function of SDC4" may be any as long as it suppresses a function (e.g., signal transduction via ligand FGF2, etc.) contributing to the cancer stem cell property of SDC4 once functionally produced. For example, a substance that binds to SDC4 to suppress the aforementioned function, a substance that inhibits the binding activity between SDC4 and a ligand, a substance that inhibits the transfer of SDC4 to the cell membrane, and the like can be mentioned.

Specifically, the substance that suppresses the function of SDC4, for example, an antibody against SDC4 can be mentioned. The antibody may be any of polyclonal antibody and monoclonal antibody. These antibodies can be produced according to a production method known per se of antibody or antiserum. While the isotype of the antibody of the present invention is not particularly limited, it is preferably IgG, IgM or IgA, particularly preferably IgG. The antibody is not particularly limited as long as it has at least a complementarity determining region (CDR) for specifically recognizing and binding to SDC4; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like, and the like.

In one preferable embodiment, an antibody against SDC4 is used as a pharmaceutical product for human as the subject of administration. Therefore, the antibody (preferably monoclonal antibody) is an antibody with a reduced risk of antigenicity when administered to human, specifically, complete human antibody, humanized antibody, mouse-human chimeric antibody or the like, particularly preferably complete human antibody. A humanized antibody and a chimeric antibody can be produced by genetic engineering by a conventional method. A complete human antibody can also be produced from human-human (or mouse) hybridoma; however, it is desirably produced using a human antibody producing mouse or phage display method in order to provide a large amount of antibody stably at a low cost.

The substance that suppresses the function of SDC4 may also be, for example, an antagonist that competitively binds to SDC4 with the ligand FGF2. Such antagonist can be obtained by constructing a competitive assay system using SDC4 and FGF2, and screening a compound library.

As shown in the below-mentioned Examples, a substance that suppresses the expression or function of SDC4 has an effect of specifically acting on and killing cancer stem cells, and is thus useful for permanent cure of cancer as a therapeutic drug targeting cancer cells. Also, a substance that suppresses the expression or function of SDC4 affords further advantageous effects of lower toxicity to normal cells and low risk of side effects. Therefore, a medicament containing a substance that suppresses the expression or function of SDC4 can be used as a drug for eliminating cancer stem cells, and, in turn, as a therapeutic drug for cancer.

Only one kind of a substance that suppresses the expression or function of SDC4 may be used or two or more kinds thereof may be used in combination. Two or more kinds of the substances that suppress the expression or function of SDC4 may be formulated as separate medicaments, or may be blended in the same pharmaceutical composition. When two or more kinds of the substances that suppress the expression or function of SDC4 are formulated as separate medicaments, the respective formulations may be simultaneously administered, or administered with time intervals. The administration routes may be the same or different. The below-mentioned doses are those of one kind of the substance that suppresses the expression or function of SDC4. Even when two or more kinds of the substances are used in combination, the same dose can be used for each substance as long as it does not adversely affect the subject of administration.

### (1) Medicament containing antisense nucleic acid, ribozyme nucleic acid, siRNA and precursor thereof

The antisense nucleic acid (or miRNA) of the present invention that can complementarily bind to a transcription product of SDC4 gene and suppress translation of protein from the transcription product, siRNA (or ribozyme, miRNA) that can cleave the transcription product by targeting a homologous (or complementary) base sequence in a transcription product of SDC4 gene, and further, shRNA, pre-miRNA and the like which are precursors of the siRNA and miRNA (hereinafter sometimes to be comprehensively referred to as "the nucleic acid of the present invention") can be used as a drug for eliminating cancer stem cells, and, in turn, as a therapeutic drug for cancer.

The medicament containing the nucleic acid of the present invention can be administered as a liquid as it is, or as an appropriate dosage form of pharmaceutical composition, to humans or non-human warm-blooded animals (e.g., rat, rabbit, sheep, swine, bovine, cat, dog, monkey, chicken and the like) orally or parenterally (e.g., intravascular administration, subcutaneous administration and the like).

When the nucleic acid of the present invention is used as a drug for eliminating cancer stem cells, it can be formulated and administered according to a method known per se. That is, the nucleic acid of the present invention may be used alone, or may also be inserted in an operative manner into a suitable expression vector for mammalian cells such as retrovirus vector, adenovirus vector, adeno-associated virus vector or the like. The nucleic acid can be administered as it is, or by a gene gun or a catheter such as hydrogel catheter together with an adjuvant for promoting ingestion. Alternatively, it can also be converted to an aerosol and topically administered as an inhalant into the trachea.

Furthermore, for the purpose of improving pharmacokinetics, prolonging the half-life, and improving intracellular uptake efficiency, the aforementioned nucleic acid may be formulated (injection) alone or together with a carrier such as liposome and the like and administered intravenously, subcutaneously, or the like.

The nucleic acid of the present invention can be directly administered or administered as a suitable pharmaceutical composition. The pharmaceutical composition used for the administration may contain the nucleic acid of the present invention, and a pharmacologically acceptable carrier, diluent or excipient. Such pharmaceutical composition is provided in a dosage form suitable for oral or parenteral administration.

As examples of the composition for parenteral administration, injections, suppositories, agents for intranasal administration, and the like are used; the injections may include dosage forms such as intravenous injections, subcutaneous injections, intracutaneous injections, intramuscular injections and drip infusion injections. Such an injection can be prepared according to a publicly known method. An injection can be prepared by, for example, dissolving, suspending or emulsifying the above-mentioned nucleic acid of the present invention in a sterile aqueous or oily solution in common use for injections. As examples of aqueous solutions for injection, physiological saline, an isotonic solution containing glucose or another auxiliary drug, and the like can be used, which may be used in combination with an appropriate solubilizer, for example, alcohol (e.g., ethanol), polyalcohol (e.g., propylene glycol, polyethylene glycol), non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with benzyl benzoate, benzyl alcohol and the like as solubilizers. The prepared injection solution is preferably filled in an appropriate ampoule. Suppositories used for rectal administration may be prepared by mixing the above-mentioned nucleic acid with an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a publicly known method, and may contain a carrier, diluent or excipient in common use in the field of pharmaceutical making. As examples of the carrier or excipient for tablets, lactose, starch, sucrose, magnesium stearate and the like can be used.

The above-mentioned parenteral or oral pharmaceutical composition is conveniently prepared in a dosage form with a dosage unit compatible with the dose of the active ingredient. Examples of the dosage form with a dosage unit include tablet, pill, capsule, injection (ampoule) and suppository. For example, the nucleic acid of the present invention is preferably contained in generally about 0.01 - 500 mg per unit dosage form.

While the dose of the above-mentioned medicament containing the nucleic acid of the present invention varies depending on the subject of administration, target illness, symptom, administration route and the like, for example, when it is used to eliminate cancer stem cells in the treatment or recurrence prevention of cancer, a single dose of the nucleic acid of the present invention is generally about 0.0001 - 20 mg/kg body weight, which is conveniently administered once a day - about once in 6 months by intravenous injection. In the case of other parenteral administration and oral administration, an amount analogous thereto can be administered. When the symptom is particularly severe, the dose may be increased according to the symptom.

Each of the aforementioned compositions may contain any other active ingredients that do not produce an unwanted interaction when formulated with the nucleic acid of the present invention. As such other active ingredient, various compounds having a treatment effect on cancer can be blended as appropriate. For example, as other active ingredients, alkylating agents (e.g., mustards, nitrosoureas), metabolic antagonists (e.g., folic acid, pyrimidine, purine), antitumor antibiotics (e.g., anthracycline), hormone-like drugs (e.g., anti-estrogen drugs, anti-androgen drugs, LH-RH agonists, progesterone, estradiol), platinum preparations, topoisomerase inhibitors (e.g., topoisomerase I inhibitors, topoisomerase II inhibitors), biologics (e.g., interferon, interleukin), molecular targeting drugs (e.g., antibodies (e.g., trastuzumab, panitumumab), low-molecular-weight molecules (gefinitib, errotinib, sorafenib), retinoin) and the like may be optionally contained. Particularly, chemotherapy drugs (e.g., gemcitabine, oxaliplatin etc.), Wnt inhibitors (e.g., FH535, PNU, IWR etc.), and mTOR inhibitors (e.g., rapamycin etc.) can exhibit a superior antitumor cell effect on cancer stem cells when used in combination with the nucleic acid of the present invention.

### (2) Medicament containing antibody against SDC4, low-molecular-weight compound that suppresses expression or function of SDC4 and the like

An antibody against SDC4 and a low-molecular-weight compound that suppresses the expression or function of SDC4 can inhibit production of SDC4 or function thereof contributing to the property of cancer stem cells. Therefore, these substances can be used as a drug for eliminating cancer stem cells, and, in turn, as a therapeutic drug for cancer.

A medicament containing the above-mentioned antibody or low-molecular-weight compound can be administered as a liquid as it is, or as an appropriate dosage form of pharmaceutical composition, to humans or other warm-blooded mammals (e.g., rat, rabbit, sheep, swine, bovine, cat, dog, monkey, chicken and the like) orally or parenterally (e.g., intravascular administration, subcutaneous administration and the like).

The above-mentioned antibody or low-molecular-weight compound can be directly administered or administered as a suitable pharmaceutical composition. The pharmaceutical composition used for the administration may contain the above-mentioned antibody or low-molecular-weight compound or a salt thereof, and a pharmacologically acceptable carrier, diluent or excipient. Such pharmaceutical composition is provided in a dosage form suitable for oral or parenteral administration.

As examples of the composition for parenteral administration, injections, suppositories, intranasal administration agents, and the like are used; the injections may include dosage forms such as intravenous injections, subcutaneous injections, intracutaneous injections, intramuscular injections and drip infusion injections. Such an injection can be prepared according to a publicly known method. An injection can be prepared by, for example, dissolving, suspending or emulsifying the above-mentioned antibody or low-molecular-weight compound or a salt thereof of the present invention in a sterile aqueous or oily solution in common use for injections. As examples of aqueous solutions for injection, physiological saline, an isotonic solution containing glucose or another auxiliary drug, and the like can be used, which may be used in combination with an appropriate solubilizer, for example, alcohol (e.g., ethanol), polyalcohol (e.g., propylene glycol, polyethylene glycol), non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with benzyl benzoate, benzyl alcohol and the like as solubilizers. The prepared injection solution is preferably filled in an appropriate ampoule. Suppositories used for rectal administration may be prepared by mixing the above-mentioned antibody or a salt thereof with an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a publicly known method, and may contain a carrier, diluent or excipient in common use in the field of pharmaceutical making. As examples of the carrier or excipient for tablets, lactose, starch, sucrose, magnesium stearate and the like can be used.

The above-mentioned parenteral or oral pharmaceutical composition is conveniently prepared in a dosage form with a dosage unit compatible with the dose of the active ingredient. Examples of the dosage form with a dosage unit include tablet, pill, capsule, injection (ampoule) and suppository. The antibody or low-molecular-weight compound is preferably contained in generally 0.1 - 500 mg per dosage form with a dosage unit, particularly 5 - 100 mg for injection and 10 - 250 mg for other dosage form.

While the dose of the above-mentioned medicament containing the above-mentioned antibody or low-molecular-weight compound or a salt thereof varies depending on the subject of administration, target illness, symptom, administration route and the like, for example, a single dose of the antibody or low-molecular-weight compound is generally about 0.0001 - 20 mg/kg body weight, which is orally or parenterally administered about 1 - 5 times per day when it is the low-molecular-weight compound, or the like, or once a day - once in several months by intravenous injection when it is the antibody. In the case of other parenteral administration and oral administration, an amount analogous thereto can be administered. When the symptom is particularly severe, the dose may be increased according to the symptom.

Each of the aforementioned compositions may contain any other active ingredients that do not produce an unwanted interaction when formulated with the above-mentioned antibody and low-molecular-weight compound. For example, as other active ingredients, alkylating agents (e.g., mustards, nitrosoureas), metabolic antagonists (e.g., folic acid, pyrimidine, purine), antitumor antibiotics (e.g., anthracycline), hormone-like drugs (e.g., anti-estrogen drugs, anti-androgen drugs, LH-RH agonists, progesterone, estradiol), platinum preparations, topoisomerase inhibitors (e.g., topoisomerase I inhibitors, topoisomerase II inhibitors), biologics (e.g., interferon, interleukin), molecular targeting drugs (e.g., antibodies (e.g., trastuzumab, panitumumab), low-molecular-weight molecules (gefinitib, errotinib, sorafenib), retinoin) and the like may be optionally contained.

The present invention also provides a method for detecting or sorting a cancer stem cell in a cancer cell population, including using expression of SDC4 as an index (hereinafter to be also referred to as "the detection and sorting method of the present invention").

As shown in the below-mentioned Examples, SDC4 is a cancer stem cell marker superior to CD44v9, which is a known cancer stem cell marker. Therefore, cancer stem cells can be specifically detected and sorted from a cancer cell population by using the expression of SDC4 as an index.

The type of cancer of the cancer stem cell that can be detected and sorted by the detection and sorting method of the present invention is not particularly limited and any cancer can be mentioned. For example, it may be a cancer derived from epithelial cells or may be non-epithelial sarcoma or hematologic cancer. More specifically, for example, it includes, but is not limited to, gastrointestinal cancer (e.g., esophageal cancer, stomach cancer, duodenum cancer, colorectal cancer (colon cancer, rectal cancer), liver cancer (hepatocellular cancer, cholangiocellular cancer), gall bladder cancer, bile duct cancer, pancreatic cancer, anal cancer), urinary organ cancer (e.g., kidney cancer, ureteral cancer, bladder cancer, prostate cancer, penile cancer, testis (orchis) cancer), thorax cancer (e.g., breast cancer, lung cancer (non-small cell lung cancer, small cell lung cancer)), cancer of reproductive organ(e.g., uterine cancer (uterus cervix cancer, cancer of the uterine body), ovarian cancer, vulvar cancer, vaginal cancer), head and neck cancer (e.g., maxilla cancer, pharyngeal cancer, laryngeal cancer, tongue cancer, thyroid cancer), skin cancer (e.g., basal cell carcinoma, squamous cell carcinoma), and mesothelial cell cancer (mesothelioma). Preferably, colorectal cancer, pancreatic cancer, esophageal cancer, mesothelioma, breast cancer, stomach cancer, prostate cancer and the like, more preferably colorectal cancer, pancreatic cancer, esophageal cancer, mesothelioma and the like, can be mentioned.

In the detection and sorting method of the present invention, the expression of SDC4 can be detected preferably using an antibody against SDC4 and various immunochemical methods. As the antibody against SDC4, an antibody used as an active ingredient of the aforementioned cancer stem cell elimination agent can be used similarly. For this purpose, a whole antibody molecule may be used, and any fragment thereof, such as the F(ab')₂, Fab' or Fab fraction of the antibody molecule, may also be used.

The cancer cell population to be the target of the detection and sorting of cancer stem cells is not particularly limited and may be, for example, a cancer tissue excised from a patient, a cancer cell line induced from a cancer tissue, or the like.

As the labeling agent to be used for the measurement method using a labeling substance, for example, a radioisotope, an enzyme, a fluorescent substance, a luminescent substance and the like can be used. As the radioisotope, for example, [¹²⁵I], [¹³¹I], [³H], [¹⁴C] or the like can be used. The above-described enzyme is preferably stable and has a high specific activity and, for example, β-galactosidase, β-glucosidase, alkaline phosphatase, peroxidase, malate dehydrogenase and the like can be used. As the fluorescent substance, for example, fluorescamine, fluorescein isothiocyanate (FITC), phycoerythrin (PE) and the like can be used. As the luminescent substance, for example, luminol, luminol derivative, luciferin, lucigenin and the like can be used.

The antibody of the present invention may be directly or indirectly labeled with a labeling agent. In a preferable embodiment, the anti-SDC4 antibody is an unlabeled antibody and SDC4 can be detected by the labeled secondary antibody such as anti-serum or anti-Ig antibody against the animal from which the anti-SDC4 antibody was produced. Alternatively, the biotinylated secondary antibody can be used and a complex of SDC4-anti-SDC4 antibody-secondary antibody can be formed and visualized using a labeled streptavidin.

For example, a test cancer cell sample can be fixed with glutaraldehyde, paraformaldehyde or the like and permeabilized, washed with a buffer such as PBS, blocked with BSA or the like and incubated with an anti-iPS/ES cell antibody of the present invention. After washing with a buffer such as PBS to remove unreacted antibody, the cells reacted with the anti-SDC4 antibody can be visualized with the labeled secondary antibody and analyzed using a confocal laser scanning microscope, an automated live cell image analyzer such as IN Cell Analyzer (Amarsham/GE) and the like.

In another embodiment, the anti-SDC4 antibody is used to isolate cancer stem cells from a cancer cell population containing cancer stem cells. For this purpose, for example, the anti-SDC4 antibody may be immobilized on a solid phase containing any suitable matrix such as agarose, acrylamide, Sepharose, Sephadex and the like. The solid phase may also be any suitable culture vessel such as a microtiter plate. Cancer stem cells in a sample are immobilized on the solid phase when the sample is brought into contact with the solid phase. The cells can be released from the solid phase using an appropriate elution buffer.

In another embodiment, the anti-SDC4 antibody is immobilized on magnetic beads such that cancer stem cell can be separated from the sample upon provision of a magnetic field (i.e., magnetic activated cell sorting; MACS). In still another preferable embodiment, the anti-SDC4 antibody is directly or indirectly labeled with any suitable fluorescent molecule as exemplified above and cancer stem cells can be isolated using a fluorescence activated cell sorter (FACS).

The present invention also provides a reagent for detecting a cancer stem cell, containing an antibody against SDC4. As the anti-SDC4 antibody used here, an antibody used as the active ingredient of the aforementioned cancer stem cell elimination agent can also be used similarly. As the antibody, a complete antibody molecule may be used, and any fragment, such as the F(ab')₂, Fab' or Fab fraction of the antibody molecule, may also be used. The anti-SDC4 antibody can be dissolved in an appropriate buffer known per se and preserved by freezing. It is also possible to provide a kit by combining an anti-SDC4 antibody, as well as a labeled secondary antibody, a reaction buffer, a blocking solution, washing solution and the like.

The present invention is explained in detail in the following by referring to Examples, which are not to be construed as limitative.

### [Example]

### Example 1 Search for novel cancer stem cell marker

### (1) Single cell gene analysis

A retrovirus vector encoding a fusion protein of fluorescent protein ZsGreen and ornithine decarboxylase-degron under the control of CMV promoter was introduced into human pancreatic cancer cell line Panc-1 (ATCC CRL1469) to visualize hypometabolic cells. Using anti-CD44v9 antibody, Anti human CD44v9 (obtained from Cosmo Bio; Mouse Anti-Rat IgG2a (obtained from BD pharmingen) as secondary antibody), and FACS (device name: SH800Z SONY), ZsGreen negative (dZsG-)/CD44v9 negative (CD44v9-) cell population and dZsG+/CD44v9 high expression (CD44v9^{high}) cell population were sorted. Gene analysis using a next generation sequencer (device name: HiSeqIllumina) was performed on Panc-1 dZsG-/CD44v9-, Panc-1 dZsG+/CD44v9^{high} and super Panc-1 CSC (deposit number: NITE BP-02449) which is a cultured cell derived from a tumor resulting from transplantation of 150 Panc-1 dZsG+/CD44v9^{high} cells to an immunodeficient mouse. As a result of the principle component analysis, the there could be three groups genetically separated in the gene group of PC1 (70.7%)+PC2 (29.3%), and it was clarified that they are genetically different (Fig. 1-1).

The expression of Syndecan-4 (SDC4), which belongs to the Syndecan family of heparan sulfate proteoglycan, was measured and compared by single-cell gene analysis. As a result, cancer stem cell Panc-1 dZsG+/CD44v9^{high} (n=9) showed higher expression of SDC4 than non-cancer stem cell Panc-1 dZsG-/CD44v9- (n=8) of pancreatic cancer (Fold Change:4.826; P-value:0.0001). On the other hand, super Panc-1 CSC(n=13) which is the cell line derived from tumor arising from Panc-1 dZsG+/CD44v9^{high} showed decreased expression of Sdc4 (Fig. 1-2).

### (2) Expression of SDC4 protein

### (2-1) Western blotting

Western blotting was performed using the following 4 types of cells. Anti human CD44v9 (obtained from Cosmo Bio) was used as an anti-CD44v9 antibody, Mouse Anti-Rat IgG2a (obtained from BD pharmingen) was used as a secondary antibody, Syndecan-4 (5G9) sc-12766 (obtained from Santa Cruz) was used as an anti-SDC4 antibody, and Alexa Fluor 647 (obtained from invitrogen) was used as a secondary antibody.
1-1: dZsG-/CD44v9- (cultured cells from tumor arising from 1×10⁶ non-cancer stem cells)
1-2: dZsG-/CD44v9^{high} (cultured cells from tumor arising from 1×10⁶ non-cancer stem cells)
3-3: dZsG+/CD44v9- (cultured cells from tumor arising from 150 cancer stem cells)
3-4: dZsG+/CD44v9^{high} (cultured cells from tumor arising from 150 cancer stem cells)

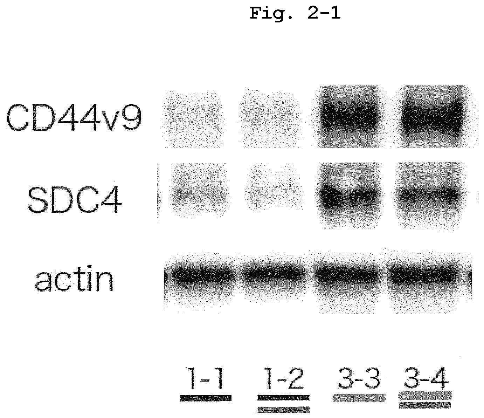

The results are shown in Fig. 2-1. The expression of SDC4 was higher in 3-3 and 3-4 than in 1-1 and 1-2, and the expression of CD44v9 was simultaneously high. It was found that the two are in parallel at a protein level.

### (2-2) Cell immunostaining

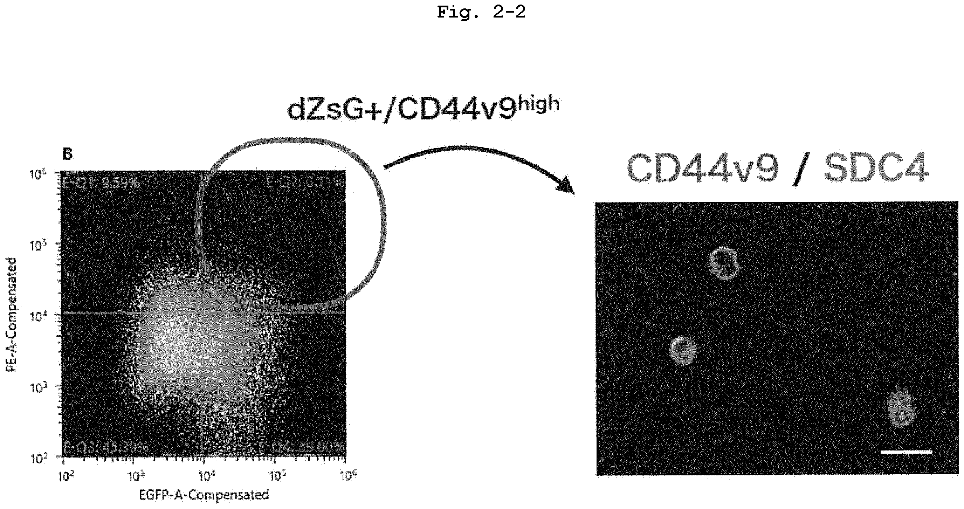

Using pancreatic cancer stem cell super Panc-1 CSC, and by a method similar to that in the above-mentioned (1), dZsG+/CD44v9^{high} cells of super Panc-1 CSC were sorted by FACS and, after 24 hr of culturing, the cells were co-stained for SDC4 and CD44v9. SDC4 was stained using an anti-SDC4 antibody (obtained from Santa Cruz) and Alexa Fluor 647 (obtained from Invitrogen) as a secondary antibody. Observation with a Keyence all-in-one fluorescence microscope (device name: BZ-X700) revealed a region where SDC4 and CD44v9 were colocalized (Fig. 2-2).

### (3) Evaluation of SDC4 as cancer stem cell marker using tumorigenicity at single cell level as index

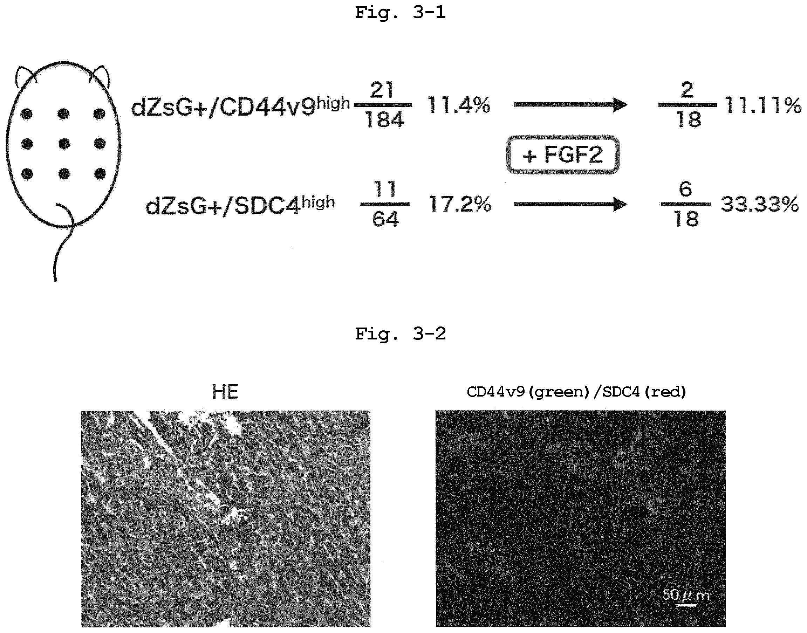

Using pancreatic cancer stem cell super Panc-1 CSC, and by a method similar to that in the above-mentioned (1), dZsG+/CD44v9^{high} cells of super Panc-1 CSC were sorted by FACS. As shown in Fig. 3-1, each one cell was subcutaneously transplanted to 9 locations on the back of SCID beige mouse (obtained from Oriental Yeast) (one cell in 50 µl DMEM+50 µl matrigel). 184 cells were subcutaneously transplanted and formation of 21 solid tumors could be confirmed 1 - 2 months later. The tumorigenicity rate thereof was 11.4% (Fig. 3-1).

On the other hand, similarly using pancreatic cancer stem cell super Panc-1 CSC, dZsG+/SDC4^{high} cells of super Panc-1 CSC were sorted by FACS. Syndecan-4 (5G9) sc-12766 (obtained from Santa Cruz) was used as an anti-SDC4 antibody, and Alexa Fluor 647 (obtained from Invitrogen) was used as a secondary antibody. Each one of the obtained cells was subcutaneously transplanted to 9 locations on the back of SCID beige mouse (one cell in 50 µl DMEM+50 µl matrigel). 64 cells were subcutaneously transplanted and formation of 11 solid tumors could be confirmed 1 - 2 months later. The tumorigenicity rate thereof was 17.2% (Fig. 3-1).

From the above, it was found that SDC4 is superior as a cancer stem cell marker to a known marker CD44v9.

Furthermore, FGF2 which is SDC4 ligand was mixed (one cell in 10 µg/ml FGF2-containing 50 µl DMEM+50 µl matrigel) and subcutaneously transplanted. As a result, the tumorigenicity rate of dZsG+/SDC4^{high} cells increased to 33.33%. On the other hand, FGF2 was mixed with dZsG+/CD44v9^{high} cells of super Panc-1 CSC and subcutaneously transplanted. As a result, the tumorigenicity rate thereof did not change and was 11.11% (Fig. 3-1) .

### (4) Tissue immunofluorescence staining of tumor arising from single cell

A tumor formed from one dZsG+/CD44v9^{high} cell of super Panc-1 CSC was subjected to tissue immunofluorescence staining for CD44v9 and SDC4. For CD44v9 staining, anti-CD44v9 antibody (obtained from Cosmo Bio; Mouse Anti-Rat IgG2a (obtained from BD pharmingen) as secondary antibody) was used; for SDC4 staining, Syndecan-4 (5G9) sc-12766 (obtained from Santa Cruz) was used as an anti-SDC4 antibody, and Alexa Fluor 647 (obtained from Invitrogen) was used as a secondary antibody. As a result, CD44v9-positive cells (green) were scarcely present but SDC4-positive cells (red) were highly frequently found (Fig. 3-2).

### (5) Evaluation of SDC4 as cancer stem cell marker using tumorigenicity by intraperitoneal administration as index

(5-1) On May 14, 2017, the following three groups of cells were sorted by FACS, 100,000 cells/1 ml DMEM were respectively administered intraperitoneally to SCID beige mice. The mice were sacrificed on July 5, 2017, and the appearance and body weight and the presence or absence of intraperitoneal tumor of the mice were examined.
(1) super Panc-1 CSC, secondary antibody alone
(2) top 4% of CD44v9^{high} of super Panc-1 CSC
(3) top 4% of SDC4^{high} of super Panc-1 CSC

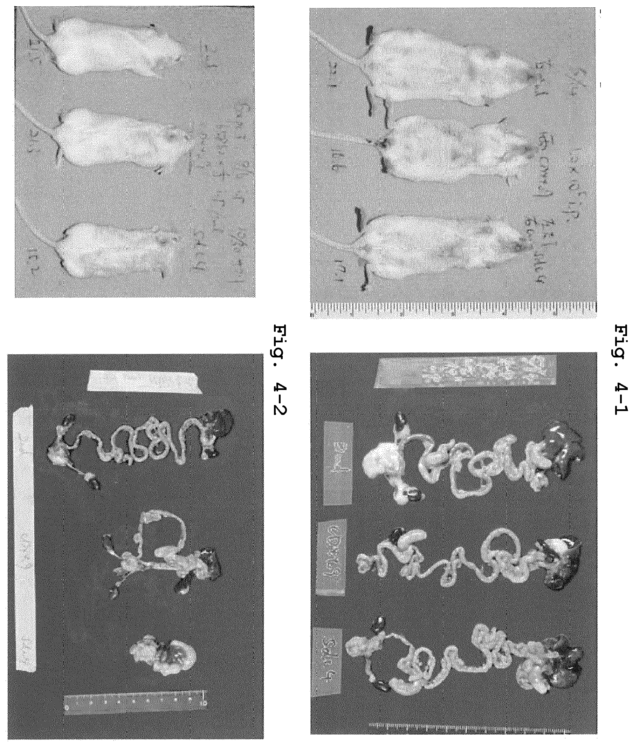

The mouse of (1) did not have special appearance to note and weighed 22.1 g, which was normal, but the mice of (2) and (3) weighed 16.6 g and 17.1 g, respectively. In particular, jaundice was observed in the mouse of (3) (Fig. 4-1, left). The mouse of (1) did not have an intraperitoneal tumor, but the mouse of (2) showed formation of several tumors around the intestinal tract. On the other hand, the mouse of (3) had a more number of solid tumors around the intestinal tract, and showed further tumorigenesis around the hepato-biliary-pancreatic area, discoloration into yellow in a part of the liver, and obvious hypertrophy of gallbladder (Fig. 4-1, right).

(5-2) On August 16, 2017, the following three groups of cells were sorted by FACS, 98,000 cells/1 ml DMEM were respectively administered intraperitoneally to SCID beige mice. The mice were sacrificed on September 19, 2017, and the appearance and body weight and the presence or absence of intraperitoneal tumor of the mice were examined.
(1) super Panc-1 CSC, secondary antibody alone
(2) top 5% of CD44v9^{high} of super Panc-1 CSC
(3) top 3.5% of SDC4^{high} of super Panc-1 CSC

The mice of (1) and (2) did not have special appearance to note and weighed 22.0 g and 22.1 g, respectively, which were normal, but the mouse of (3) weighed 15.1 g and showed jaundice. The mice of (1) and (2) did not have an intraperitoneal tumor, but the mouse of (3) showed tumorigenesis particularly around the hepato-biliary-pancreatic area, and enlargement of gallbladder.

(5-3) On August 14, 2017, the following three groups of cells were sorted by FACS, 100,000 cells/1 ml DMEM were respectively administered intraperitoneally to SCID beige mice. The mice were sacrificed on October 3, 2017, and the appearance and body weight and the presence or absence of intraperitoneal tumor of the mice were examined.
(1) super Panc-1 CSC, secondary antibody alone
(2) top 5% of CD44v9^{high} of super Panc-1 CSC
(3) top 4% of SDC4^{high} of super Panc-1 CSC

The mice of (1) and (2) did not have special appearance to note and weighed 21.2 g and 21.0 g, respectively, which were normal, but the mouse of (3) weighed 16.7 g and showed jaundice. In addition, the mice of (1) and (2) did not have an intraperitoneal tumor, but the mouse of (3) showed a solid tumor around (a part of) the intestinal tract, tumorigenesis around the hepato-biliary-pancreatic area, discoloration into yellow in a part of the liver, and obvious hypertrophy of gallbladder.

(5-4) On August 14, 2017, the following three groups of cells were sorted by FACS, 50,000 cells/1 ml DMEM were respectively administered intraperitoneally to SCID beige mice. The mice were sacrificed on September 28, 2017, and the appearance and body weight and the presence or absence of intraperitoneal tumor of the mice were examined.
(1) super Panc-1 CSC, secondary antibody alone
(2) top 5% of CD44v9^{high} of super Panc-1 CSC
(3) top 4.5% of SDC4^{high} of super Panc-1 CSC

The mice of (1) and (2) did not have special appearance to note and weighed 18.0 g and 19.4 g, respectively, which were normal, but the mouse of (3) weighed 15.0 g. The mice of (1) and (2) did not have an obvious intraperitoneal tumor, but the mouse of (3) showed tumorigenesis around the hepato-biliary-pancreatic area.

(5-5) Using different pancreatic cancer cell line Bxcp3 (ATCC CRL-1687), an experiment similar to (5-1) - (5-4) was performed. On September 6, 2017, the following three groups of cells were sorted by FACS, 21,954 cells/1 ml DMEM were respectively administered intraperitoneally to SCID beige mice. The mice were sacrificed on June 12, 2017, and the appearance and body weight and the presence or absence of intraperitoneal tumor of the mice were examined.
(1) Bxpc3, secondary antibody alone
(2) top 4% of CD44v9^{high} of Bxpc3
(3) top 5% of SDC4^{high} of Bxpc3

The mice of (1) and (2) did not have special appearance to note and weighed 21.5 g and 21.3 g, respectively, which were normal, but the mouse of (3) weighed 15.2 g (Fig. 4-2, left). The mice of (1) and (2) did not have an obvious intraperitoneal tumor, but the mouse of (3) showed tumorigenesis in the hepato-biliary-pancreatic area, intestinal obstruction and marked dilation of the stomach (Fig. 4-2, right).

### (6) Evaluation of SDC4 as cancer stem cell marker using tumorigenicity by subcutaneous injection as index

### (6-1) Panc-1

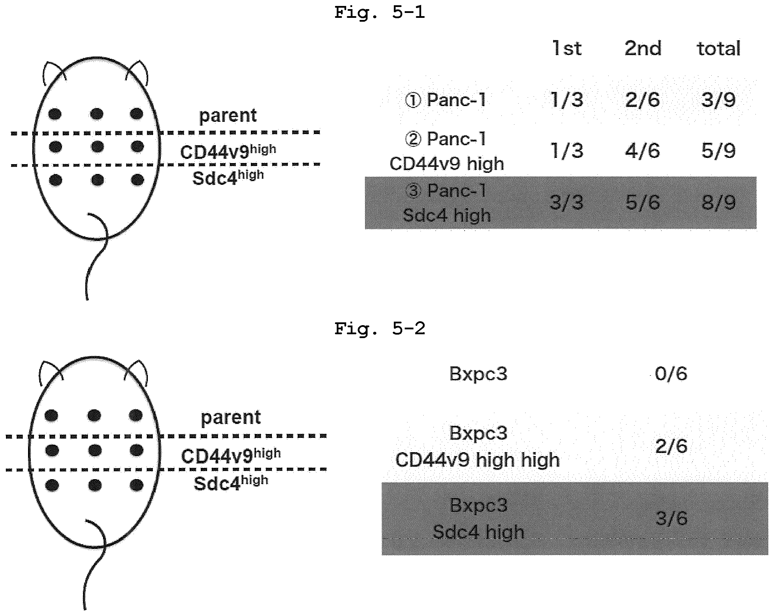

1st experiment: On January 27, 2017, the following three groups of cells were sorted by FACS, 50 cells/100 ul (DMEM:matrigel = 1:1) were respectively injected subcutaneously into the back of SCID beige mouse as shown in Fig. 5-1, and tumorigenesis up to March 21, 2017 was observed.

2nd experiment: On February 28, 2017, the following three groups of cells were sorted by FACS, 50 cells/100 ul (DMEM:matrigel = 1:1) were respectively injected subcutaneously into the back of SCID beige mouse as shown in Fig. 5-1, and tumorigenesis up to April 13, 2017 was observed.
(1) Panc-1
(2) top 8.5% of Panc-1 CD44v9^{high}
(3) top 1% of Panc-1 SDC4^{high}

The results are shown in Fig. 5-1. The cell of (3) showed the highest tumorigenicity.

### (6-2) Bxpc3

Using different pancreatic cancer cell line Bxcp3, an experiment similar to (6-1) was performed. On March 5, 2017, the following three groups of cells were sorted by FACS, 75 cells/100 ul (DMEM:matrigel = 1:1) were respectively injected subcutaneously into the back of SCID beige mouse as shown in Fig. 5-2, and tumorigenesis up to June 12, 2017 was observed.
(1) Bxpc3
(2) top 4% of Bxpc3 CD44v9^{high}
(3) top 5% of Bxpc3 SDC4^{high}

The results are shown in Fig. 5-2. The cells of (3) showed the highest tumorigenicity.

### (7) Tissue immunofluorescence staining for CD44v9 and SDC4 in cancer tissue excised from colorectal cancer patient

By a method similar to that in the above-mentioned (4), tissue immunofluorescence staining of cancer tissues excised from four colorectal cancer patients was performed. General information and pathological findings of the four samples used are shown in Table 1.

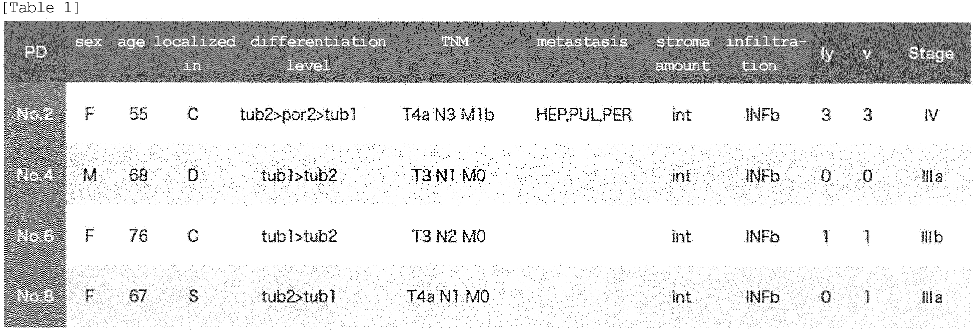

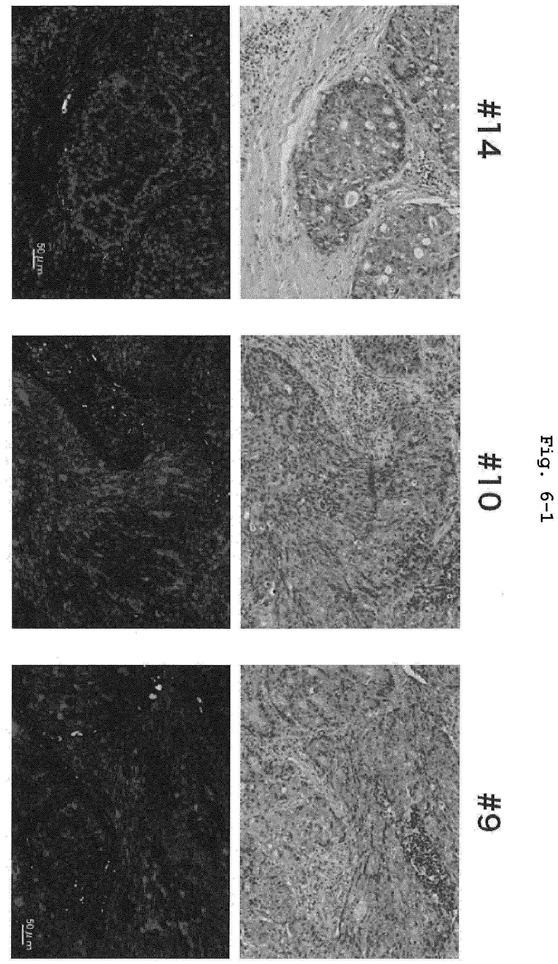

A representative example of the results of tissue fluorescence staining of sample No. 2 is shown in Fig. 6-1. SDC4 was not stained in the moderately differentiated region such as #14, but SDC4 was highly expressed in the poorly differentiated regions such as #10 and #9. However, no expression of CD44v9 was observed in the cancerous part.

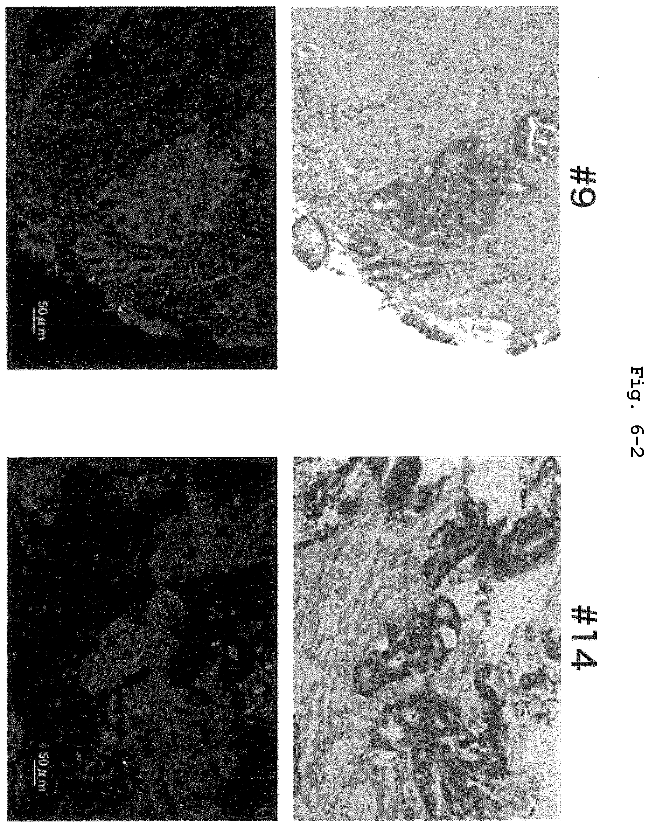

A representative example of the results of tissue fluorescence staining of sample No. 4 is shown in Fig. 6-2. SDC4 was highly expressed in the regions such as #9 and #14, but no expression of CD44v9 was observed.

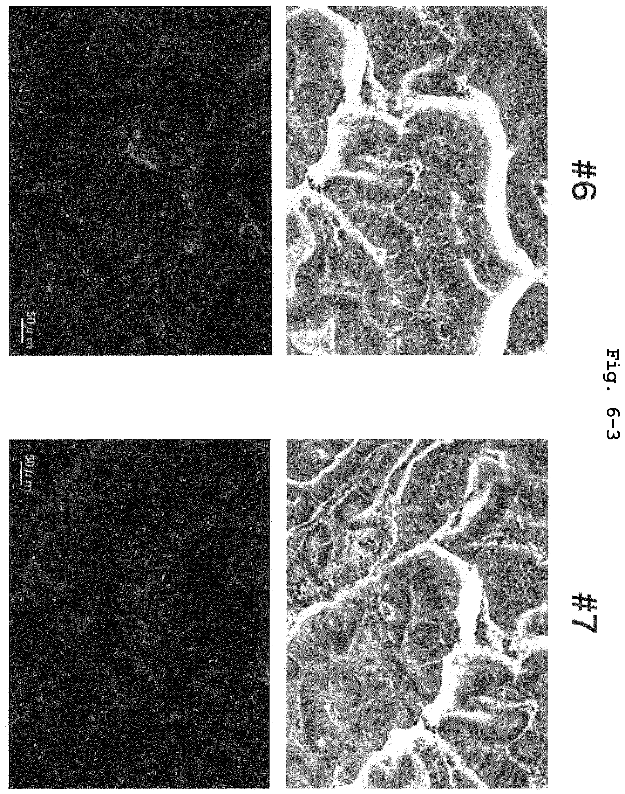

A representative example of the results of tissue fluorescence staining of sample No. 6 is shown in Fig. 6-3. In the regions of #6, #7 and the like, high co-expression of CD44v9 and SDC4 was found in the cell membrane.

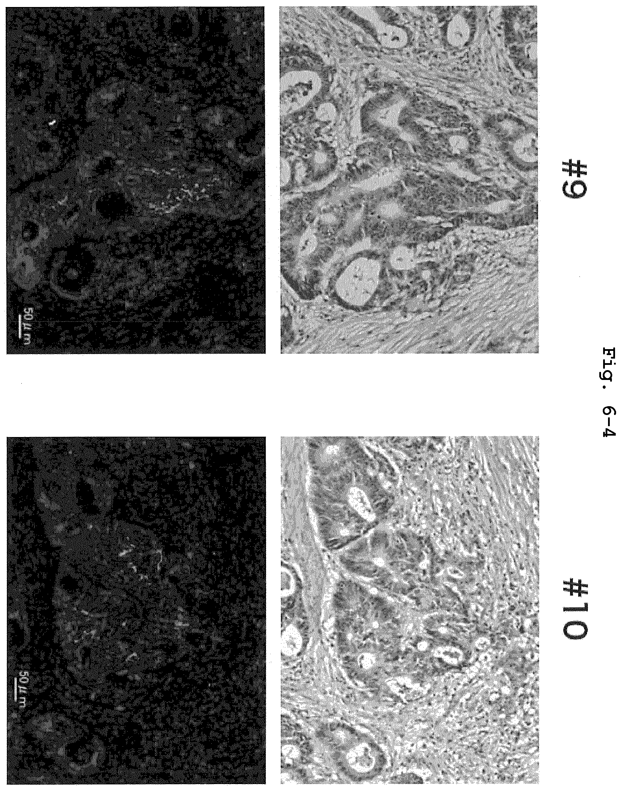

A representative example of the results of tissue fluorescence staining of sample No. 8 is shown in Fig. 6-4. In #9 and #10, the overall expression of SDC4 was high, and high co-expression of CD44v9 and SDC4 in the cell membrane was found in some part.

### (8) Tissue immunofluorescence staining for CD44v9 and SDC4 in PDX model of cancer tissue excised from colorectal cancer patient

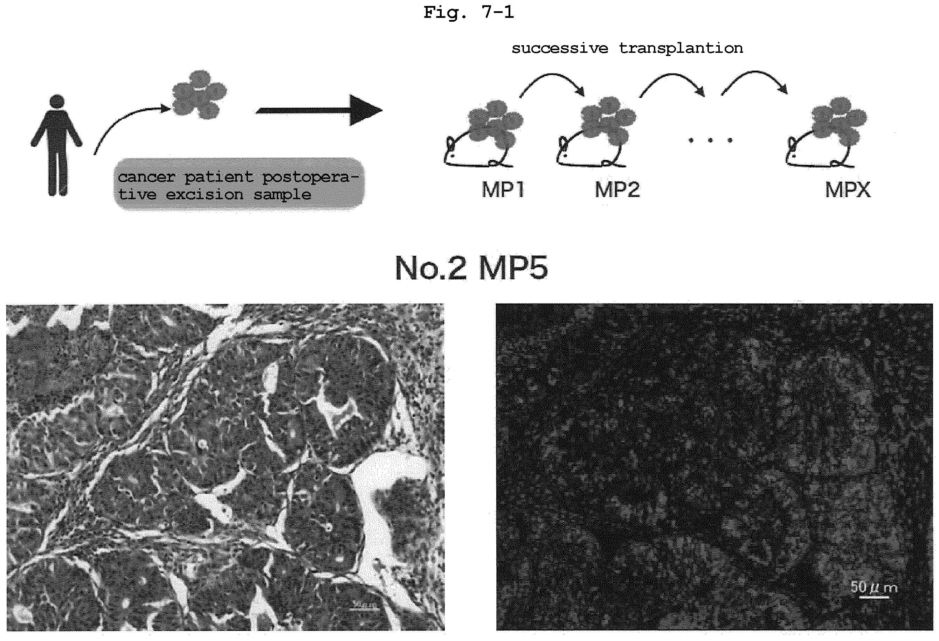

(8-1) The cancer tissue excised from sample No. 2 was transplanted to SCID beige mouse as shown in Fig. 7-1, and immunofluorescence staining was performed in the same manner as in the above-mentioned (7) using a tumor sample (MP5) successively transplanted 5 times. As a result, SDC4 was highly expressed in the cancer regions, but no expression of CD44v9 was observed (Fig. 7-1).

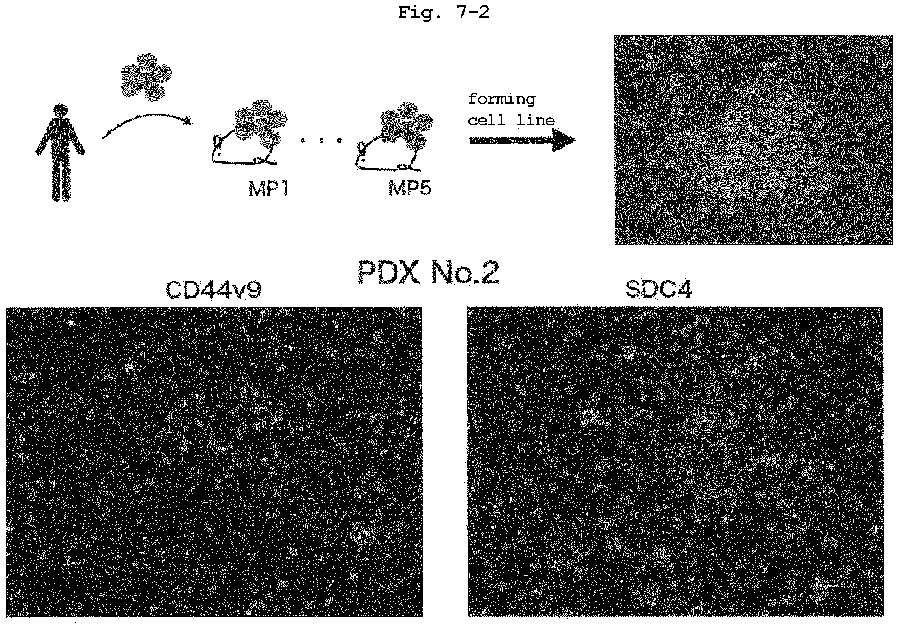

(8-2) Cell line was formed using PDX tumor sample (MP5) of cancer tissue excised from sample No. 2. Immunofluorescence staining of the obtained cell line PDX No. 2 was performed in the same manner as in the above-mentioned (7). As a result, SDC4 was highly expressed, but no expression of CD44v9 was observed (Fig. 7-2).

### (9) Evaluation of SDC4 as cancer stem cell marker using tumorigenicity using PDX No. 2 as index

(9-1) On September 10, 2017, the following two groups of cells were sorted by FACS, 10,000 cells/1 ml DMEM were respectively administered intraperitoneally to SCID beige mice. The mice were sacrificed on December 2, 2017, and the appearance and body weight and the presence or absence of intraperitoneal tumor of the mice were examined.
(1) bottom 12% of SDC4 staining of PDX No.2
(2) top 1.2% of SDC4 staining of PDX No.2

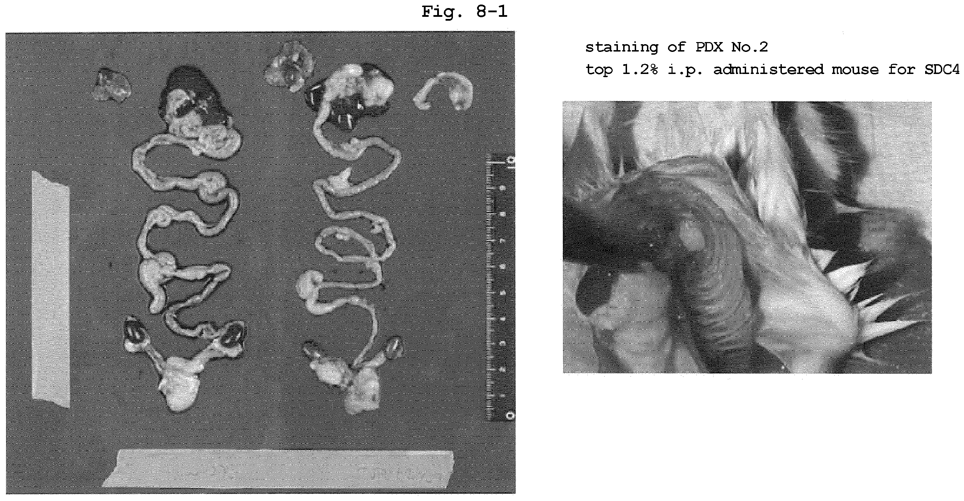

The mice of (1) and (2) did not have special appearance to note and weighed 22.9 g and 21.4 g, respectively. However, the mouse of (2) had an obvious intraperitoneal tumor in the abdominal cavity, and showed tumorigenesis around the hepato-biliary-pancreatic area, in the diaphragm, and further in the pleura of the inner wall side of the_thoracic cavity (Fig. 8-1).

(9-2) On January 30, 2018, the following three groups of cells were sorted by FACS, 10,000 cells/800 ul DMEM were respectively administered intraperitoneally to SCID beige mice (male). The mice were sacrificed on March 21, 2018, and the appearance and body weight and the presence or absence of intraperitoneal tumor of the mice were examined.
(1) bottom 12% of SDC4 staining of PDX No.2
(2) top 1% of SDC4 staining of PDX No.2
(3) PDX No.2, secondary antibody alone

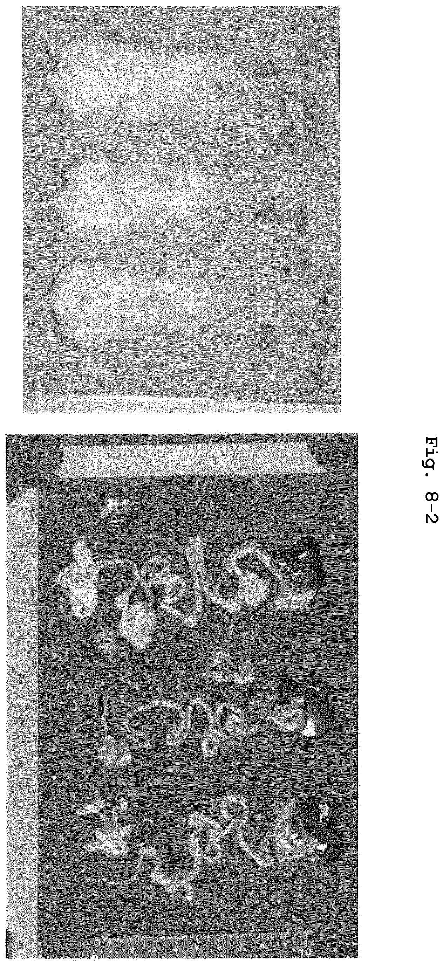

The mice of (1) and (3) did not have special appearance to note and weighed 29.6 g and 23.2 g, respectively. On the other hand, the mouse of (2) weighed 20.3 g, and showed jaundice (Fig. 8-2, left). In addition, the mouse of (2) showed tumorigenesis in a part around the intestinal tract, clear tumorigenesis around the hepato-biliary-pancreatic area, discoloration into yellow of a part of the liver, and hypertrophy of gallbladder. The mouse of (3) showed tumorigenesis in a part of the hepato-biliary-pancreatic area only. The mouse of (1) did not show tumorigenesis (Fig. 8-2, right).

### (10) Evaluation of SDC4 as cancer stem cell marker using tumorigenicity using esophageal cancer cell line index

On March 30, 2018, the following two groups of cells were sorted by FACS, 10,000 cells/800 ul DMEM were respectively administered intraperitoneally to SCID beige mice (male). The mice were sacrificed on March 21, 2018, and the appearance and body weight and the presence or absence of intraperitoneal tumor of the mice were examined.
(1) top 2% of SDC4 positive of TE4
(2) TE4, secondary antibody alone

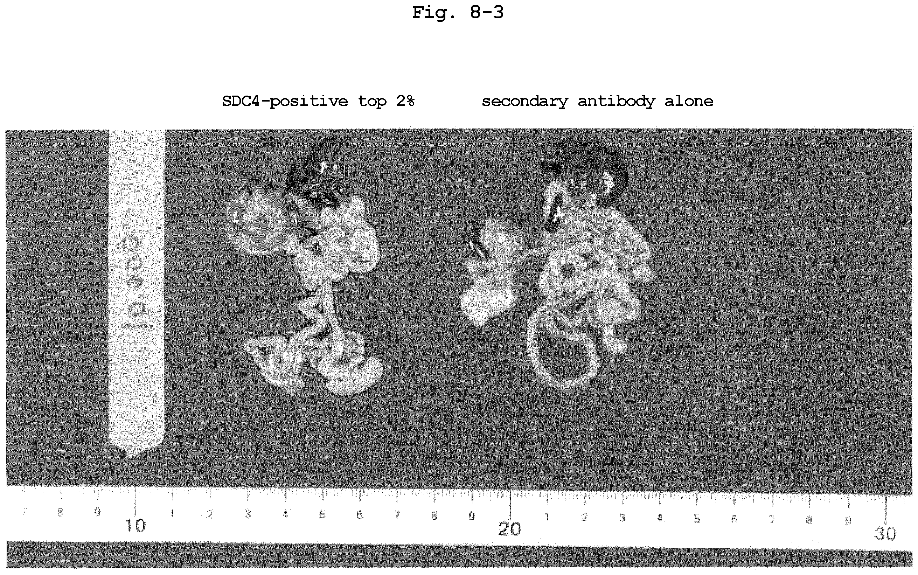

The mouse of (2) weighed 20.1 g, and the mouse of (1) weighed 17.9 g. As compared with the mouse of (2), the mouse of (1) showed tumorigenesis (Fig. 8-3).

### Example 2 Antitumor effect of siRNA targeting SDC4

### (1) Single administration

Using seven types of siRNAs including uniquely-designed 3 siRNA species #6, #7, #10 that target SDC4, commercially available siRNAs #1, #2, #3 targeting SDC4 (obtained from applied biosystems), and negative control siRNANC, the antitumor cell effect on cell line PDX No. 2 derived from the PDX model of cancer tissues excised from colorectal cancer patients (sample No. 2) and pancreatic cancer stem cell super Panc-1 CSC was verified. The nucleotide sequences of the seven types of siRNAs are shown below.
#6:(sense)5'-GGUCCUGGCAGCUCUGAUUdTdT(SEQ ID NO: 3)
   (anti-sense) 5'-AAUCAGAGCUGCCAGGACCdTdT(SEQ ID NO: 4)
#7:(sense)5'-AGGAUGAAGGCAGCUAUGAdTdT(SEQ ID NO: 5)
   (anti-sense) 5'-UCAUAGCUGCCUUCAUCCUdTdT(SEQ ID NO: 6)
#10:(sense)5'-GCAAGAAACCCAUCUACAAdTdT(SEQ ID NO: 7)
   (anti-sense) 5'-UUGUAGAUGGGUUUCUUGCdTdT(SEQ ID NO: 8)
#1:(sense)5'-CUACUGCUCAUGUACCGUAtt(SEQ ID NO: 9)
   (anti-sense) 5'-UACGGUACAUGAGCAGUAGga(SEQ ID NO: 10)
#2:(sense)5'-GCUAUGACCUGGGCAAGAAtt(SEQ ID NO: 11)
   (anti-sense) 5'-UUCUUGCCCAGGUCAUAGCtg(SEQ ID NO: 12)
#3:(sense)5'-CCAAGAAACUAGAGGAGAAtt(SEQ ID NO: 13)
   (anti-sense) 5'-UUCUCCUCUAGUUUCUUGGgt(SEQ ID NO: 14)
NC:(sense)5'-AUCCGCGCGAUAGUACGUA(SEQ ID NO: 15)
   (anti-sense) 5'-AUCCGCGCGAUAGUACGUA(SEQ ID NO: 16)

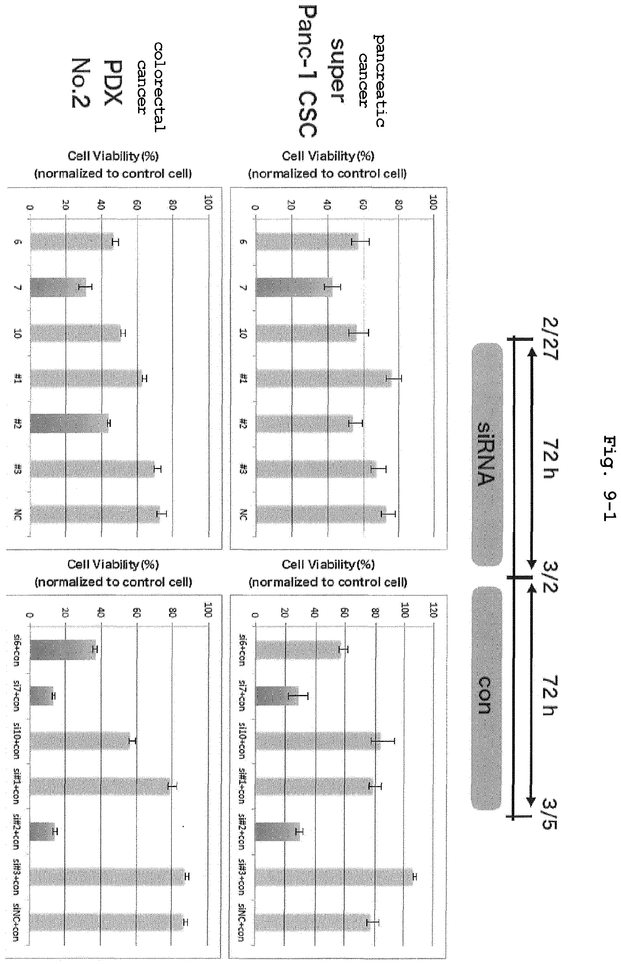

6000 cells/well were sorted by FACS into each of the 96 wells and allowed to stand overnight, then siRNA 10 pmol/well was introduced by lipofectamin2000 (obtained from Invitrogen), and after 72 hr, the cell viability was measured by WST8 (obtained from DojinDo). Furthermore, the medium was replaced with DMEM (FBS 10%) in all wells, and after 72 hr, the cell viability was measured again by WST8. After 72h+72h, siRNAs #7 and #2 showed remarkable antitumor cell effects (Fig. 9-1).

### (2) Combined administration with other antitumor drugs

### (2-1) Combined use with chemotherapeutic agent

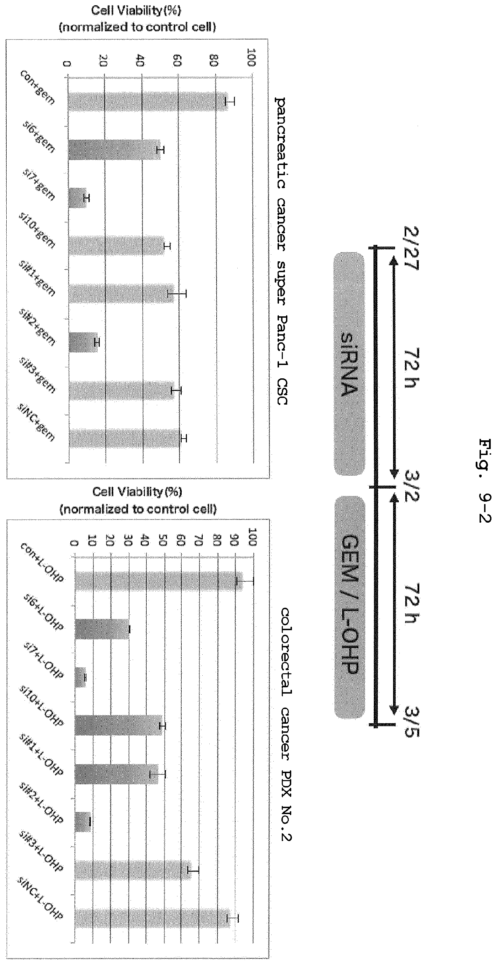

In the same manner as in the above-mentioned (1), the effect of combined use of 7 types of siRNAs and a chemotherapeutic agent was investigated. 6000 cells/well were sorted by FACS into each of the 96 wells and allowed to stand overnight, siRNA 10 pmol/well was introduced by lipofectamine2000, and after 72 hr, the cell viability was measured by WST8. Then, the medium was replaced with gemcitabine (GEM)-containing DMEM (FBS 10%) in all wells of pancreatic cancer super Panc-1 CSC, the medium was replaced with oxaliplatin (L-OHP)-containing DMEM (FBS 10%) in all wells of colorectal cancer PDX No.2, and after 72 hr, the cell viability was measured again by WST8. After 72h+72h, siRNA7+GEM and #2+GEM showed remarkable antitumor cell effects on pancreatic cancer super Panc-1 CSC, and siRNA7+L-OHP and #2+L-OHP showed remarkable antitumor cell effects on colorectal cancer PDX No.2 (Fig. 9-2).

### (2-2) Combined use with Wnt inhibitor

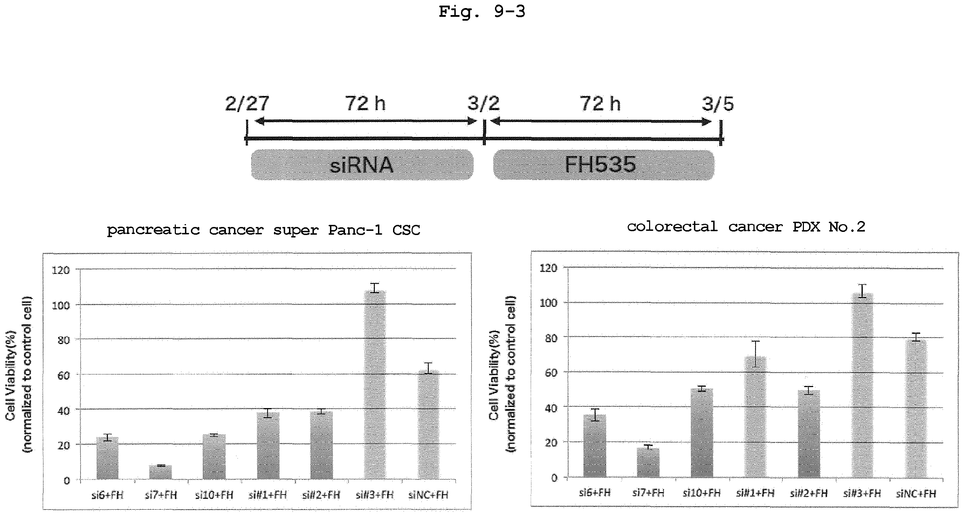

In the same manner as in the above-mentioned (1), the effect of combined use of 7 species of siRNAs and a Wnt inhibitor was investigated. 6000 cells/well were sorted by FACS into each of the 96 wells and allowed to stand overnight, siRNA 10 pmol/well was introduced by lipofectamine2000, and after 72 hr, and the cell viability was measured by WST8. Then, the medium was replaced with Wnt inhibitor (FH535)-containing DMEM (FBS 10%) in all wells, and after 72 hr, the cell viability was measured again by WST8. After 72h+72h, siRNA#7+FH535 showed remarkable antitumor cell effect (Fig. 9-3) .

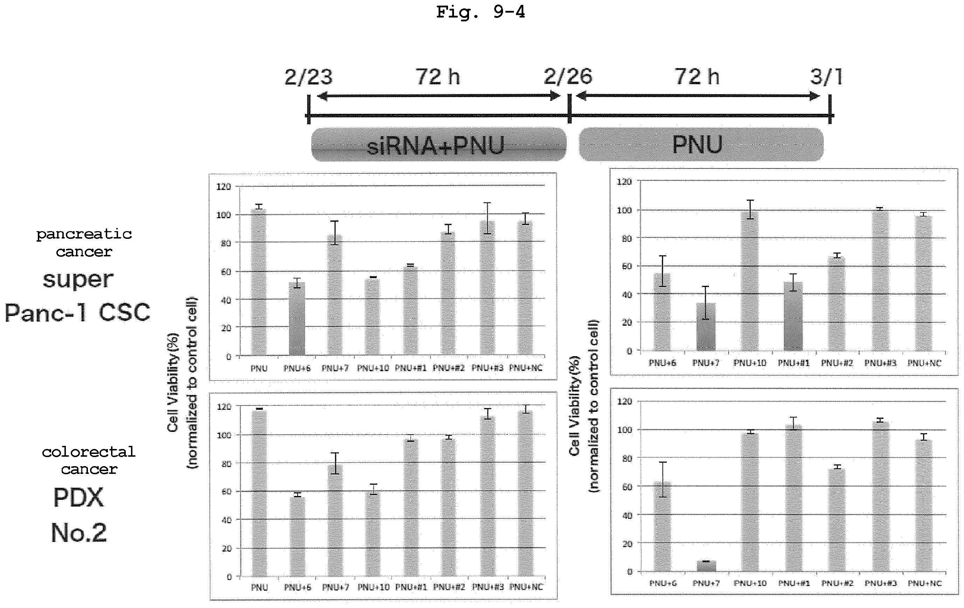

In addition, 6000 cells/well were sorted by FACS into each of the 96 wells and allowed to stand overnight, siRNA 10 pmol/well was introduced by lipofectamine2000, and a Wnt inhibitor PNU was also added simultaneously. After 72 hr, the cell viability was measured by WST8. Then, the medium was replaced with PNU-containing DMEM (FBS 10%) in all wells, and after 72 hr, the cell viability was measured again by WST8. After 72h+72h, siRNA#7+PNU showed remarkable antitumor cell effect (Fig. 9-4).

### (2-3) Combined use with mTOR inhibitor

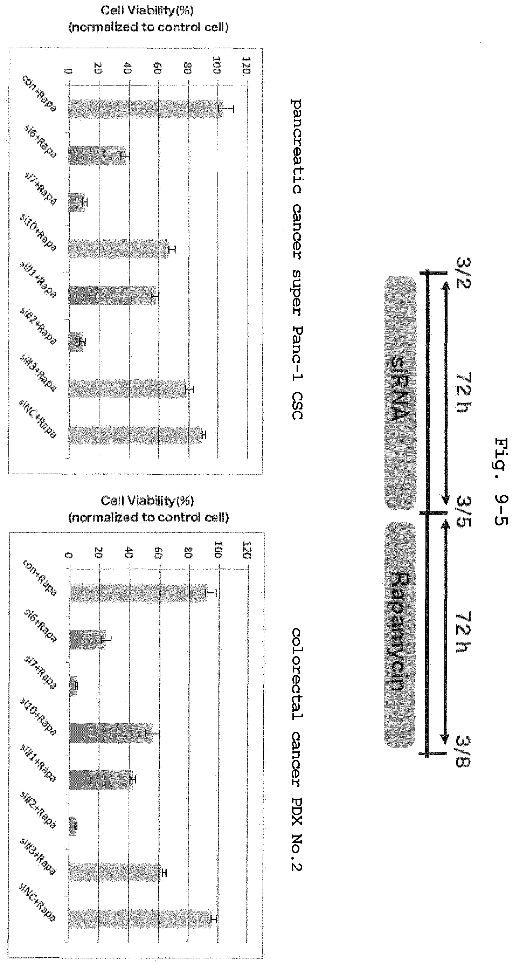

In the same manner as in the above-mentioned (1), the effect of combined use of 7 species of siRNAs and an mTOR inhibitor was investigated. 6000 cells/well were sorted by FACS into each of the 96 wells and allowed to stand overnight, siRNA 10 pmol/well was introduced by lipofectamine2000, and after 72 hr, and the cell viability was measured by WST8. Then, the medium was replaced with mTOR inhibitor (rapamycin)-containing DMEM (FBS 10%) in all wells, and after 72 hr, the cell viability was measured again by WST8. After 72h+72h, siRNA#7+rapamycin and siRNA#2+rapamycin showed remarkable antitumor cell effects (Fig. 9-5).

### (3) Toxicity of siRNA targeting SDC4 on normal cell

### (3-1) Toxicity test of siRNA targeting SDC4 to normal cell and cancer stem cell

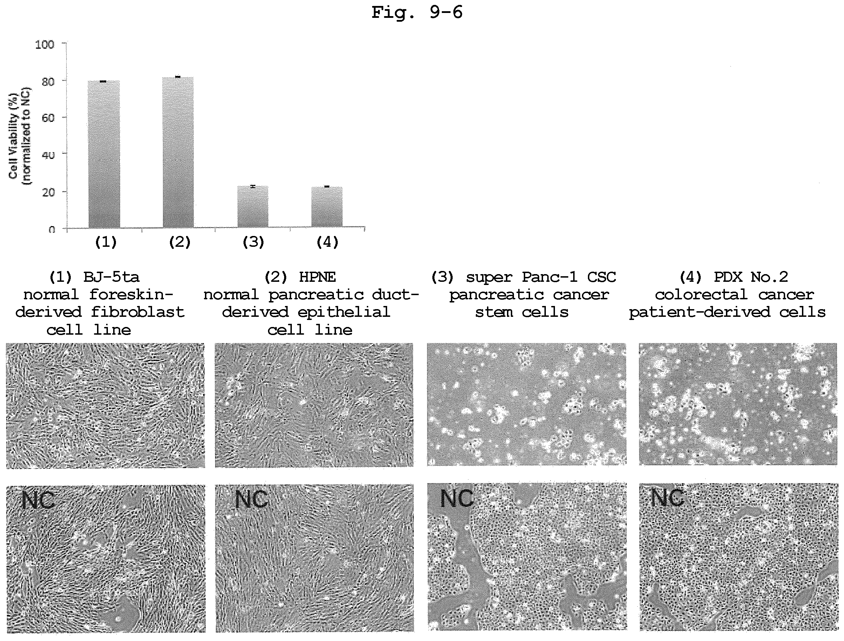

Using commercially available siRNA#2 targeting SDC4 and negative control siRNA NC, the toxicity of BJ-5ta normal foreskin-derived fibroblast line, HPNE normal pancreatic duct-derived epithelial cell line, 3-4 pancreatic cancer stem cell, and cell line PDX No.2 derived from the PDX model of cancer tissue excised from No.2 H16-2490 colorectal cancer patients was verified. 200,000 cells/well were plated on each of the 6 wells and, after culturing overnight, siRNA 100 pmol/well was introduced by lipofectamine2000, and the cell viability was measured by WST8 96 h later. As a result, toxicity to two normal cells was not found, and remarkable toxicity to two types of cancer cells was found (Fig. 9-6).

### (3-2) Expression of SDC4 in normal cell and cancer stem cell

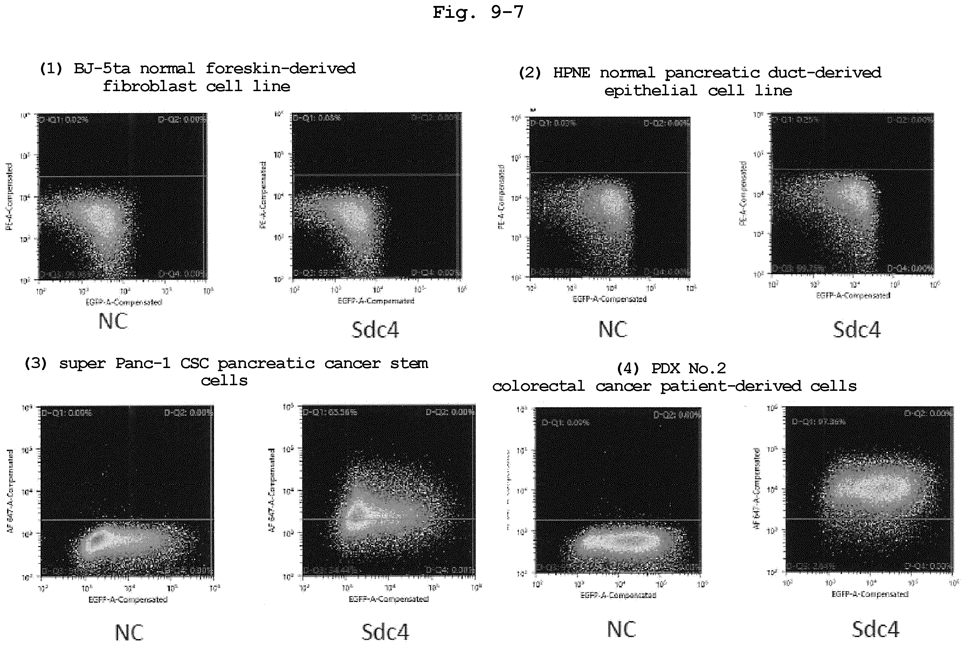

BJ-5ta normal foreskin-derived fibroblast line, HPNE normal pancreatic duct-derived epithelial cell line, super Panc-1 CSC pancreatic cancer stem cell, and cell line PDX No.2 derived from the PDX model of cancer tissue excised from No.2 H16-2490 colorectal cancer patients were subjected to cell immunostaining with SDC4 antibody, and analyzed by FACS. As a result, expression of SDC4 was not found in two normal cells, and remarkable SDC4 expression was found in two types of cancer cells (Fig. 9-7).

### (3-3) Antitumor effect of siRNA targeting SDC4 on mouse subcutaneous solid tumor model

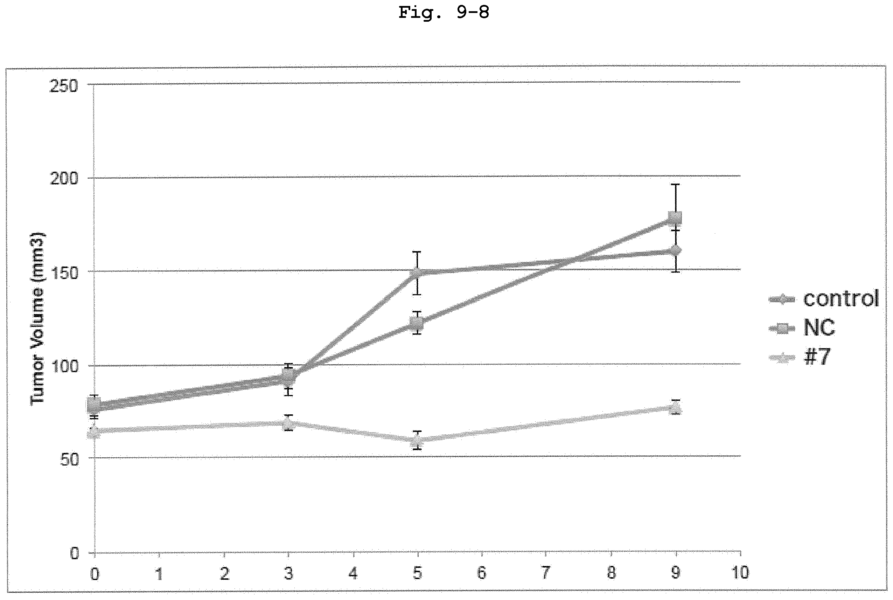

Super Panc-1 CSC pancreatic cancer stem cells were subcutaneously transplanted to a nude mouse at 3×10⁶ cells/Tumor. When the volume reached about 70 mm³, siRNA #7 targeting SDC4 or negative control siRNA NC was intravenously injected once per day using super apatite which is a nucleic acid delivery system at 20 ug/injection from the mouse tail vein. A remarkable antitumor effect was found in the #7 administration group as compared with the untreated group (control) and the negative control administration group (NC). On Day5 and Day9, the volume of the tumor was significantly small in the #7 administration group as compared with the control and NC (Fig. 9-8).

### (4) Mechanism of Antitumor effect of siRNA targeting SDC4

### (4-1) Relationship between protein level and antitumor effect of SDC4

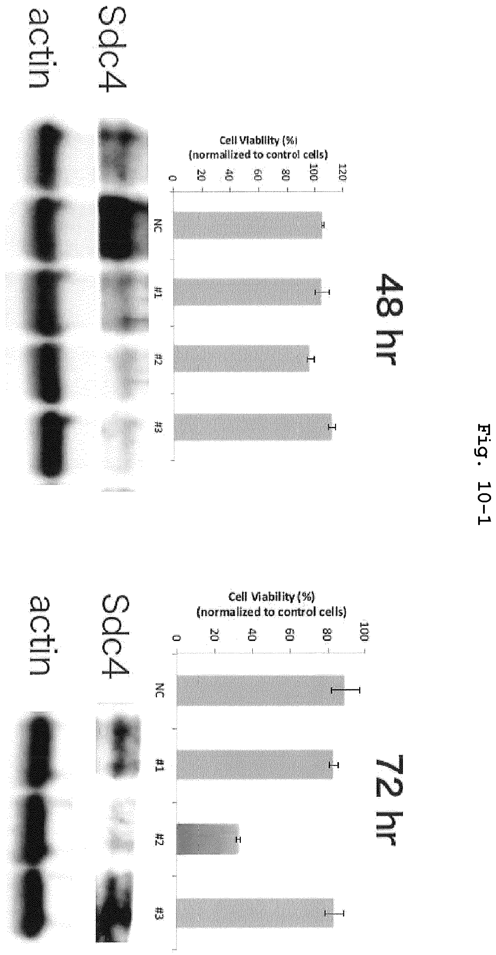

The mechanism of the antitumor cell effect of commercially available siRNAs #1, #2, #3 targeting SDC4, and negative control siRNANC on pancreatic cancer stem cell super Panc-1 CSC was verified. 6000 cells/well were sorted by FACS into each of the 96 wells and allowed to stand overnight, siRNA 10 pmol/well was introduced by lipofectamine2000, and after 48h and 72 hr, the cell viability was measured by WST8. In addition, the protein level of SDC4 was measured by Western blotting. As a result, a decrease in SDC4 protein by siRNAs #1, #2, #3 was found as compared with the negative control siRNANC at 48 hr, but there was no change in cell viability. At 72 hr, only siRNA #2 targeting SDC4 showed a decrease in SDC4 protein and cell viability (Fig. 10-1).

### (4-2) Mechanism prediction by upstream analysis of IPA

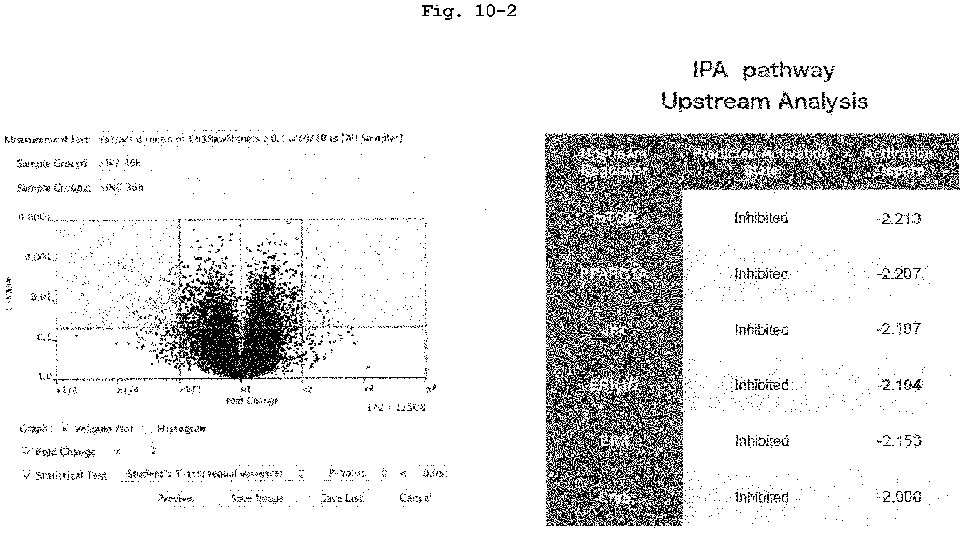

The mechanism of the antitumor cell effect of commercially available siRNA #2 targeting SDC4 on pancreatic cancer stem cell super Panc-1 CSC was verified. 6000 cells/well were sorted by FACS into each of the 96 wells and allowed to stand overnight, siRNA 10 pmol/well was introduced by lipofectamine2000. After 36 hr, the cells were collected, RNA was extracted, and RNA-sequencing was performed. As a result, there were 172 genes in siRNA #2 that varied at Fold Change 2.0 or more, p<0.05 as compared with the negative control siRNANC. This 172 variable gene set was subjected to the Upstream analysis by Ingenuity Pathways Analysis (IPA). As a result, suppression of the Upstream regulators such as mTOR, PPARG1A, Jnk, ERK1/2, ERK, Creb and the like was predicted (Fig. 10-2) .

### (4-3) Induction of SDC4 expression by Wnt inhibitor

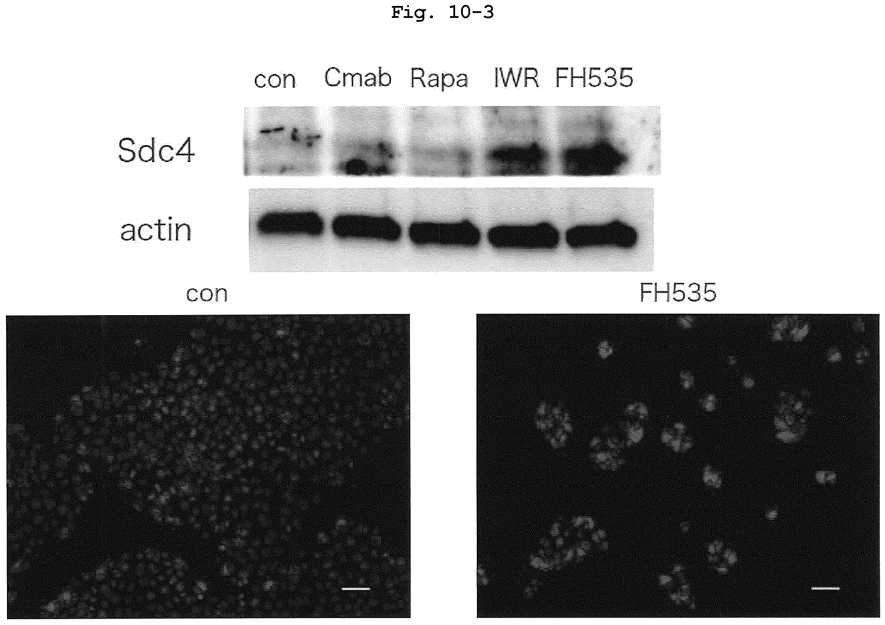

Pancreatic cancer stem cell super Panc-1 CSC was sorted by FACS in each of the 96 wells at 6000 cells/well and cultured overnight. Cetuximab (Cmab), Rapamycin, and two kinds of Wnt inhibitors (IWR, FH535) were added and, 48 hr later, SDC4 cell immunostaining and Western blotting were performed. In Western blotting, the protein expression of SDC4 increased by the addition of Wnt inhibitors IWR and FH535. Immunofluorescence cell staining also showed increased SDC4 expression by FH535 (Fig. 10-3).

### Example 3 Hypoxic region and expression of SDC4 in tumor formed from one cancer stem cell

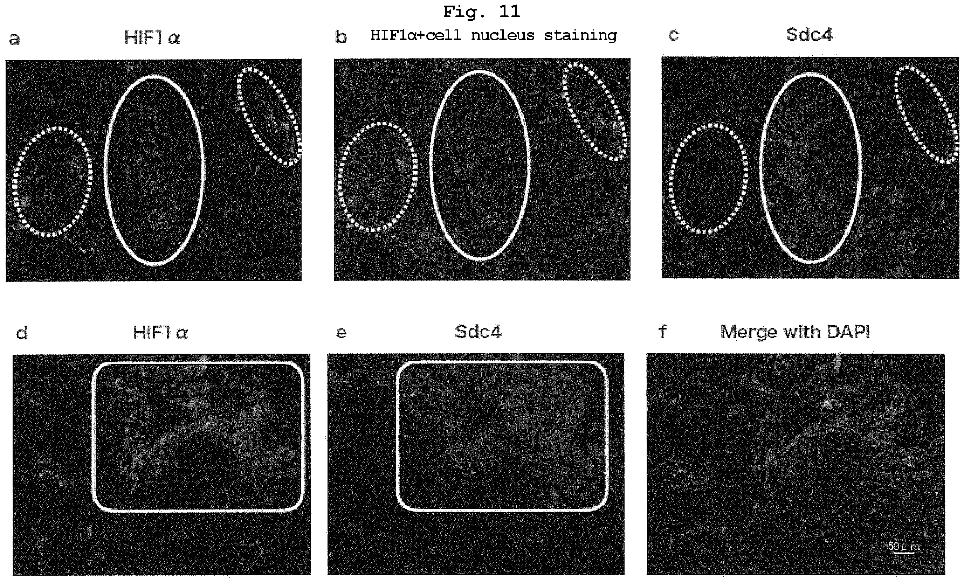

Six each (n=6) of 21 solid tumors derived from dZsG+/CD44v9^{high} single cell of super Panc-1 CSC, and 11 solid tumors derived from dZsG+/SDC4^{high} single cell of super Panc-1 CSC, which were obtained in Example 1(3), were subjected to immunofluorescence staining for HIF1α and SDC4. HIF1α stabilizes and accumulates in hypoxic conditions and migrates from the cytoplasm to the nucleus. Thus, by double staining for HIF1α and with DAPI, the region where the cell nucleus is HIF1α positive corresponds to the hypoxic region, and the region where the cell nucleus is HIF1α negative corresponds to the non-hypoxic region. The results are shown in Fig. 11. In Fig. 11(a) - (c) (derived from dZsG+/CD44v9^{high} single cell), the nucleus is HIF1α positive in the part surrounded by the white solid line, and the nucleus is HIF1α negative in the part surrounded by the white dotted line. Therefore, many SDC4(+) cells were confirmed in the hypoxic region. Also in the dZsG+/SDC4^{high} single cell-derived solid tumors (Fig. 11(d) -(f)), SDC4(+) could be similarly confirmed in the HIF1α nuclear staining positive (part surrounded by the white solid line) hypoxic region. On the other hand, the expression of SDC4 could not be confirmed in the non-hypoxic region.

### Example 4 Antitumor effect of anti-SDC4 antibody

### (1) Antitumor effect of anti-SDC4 antibody on pancreatic cancer and colorectal cancer

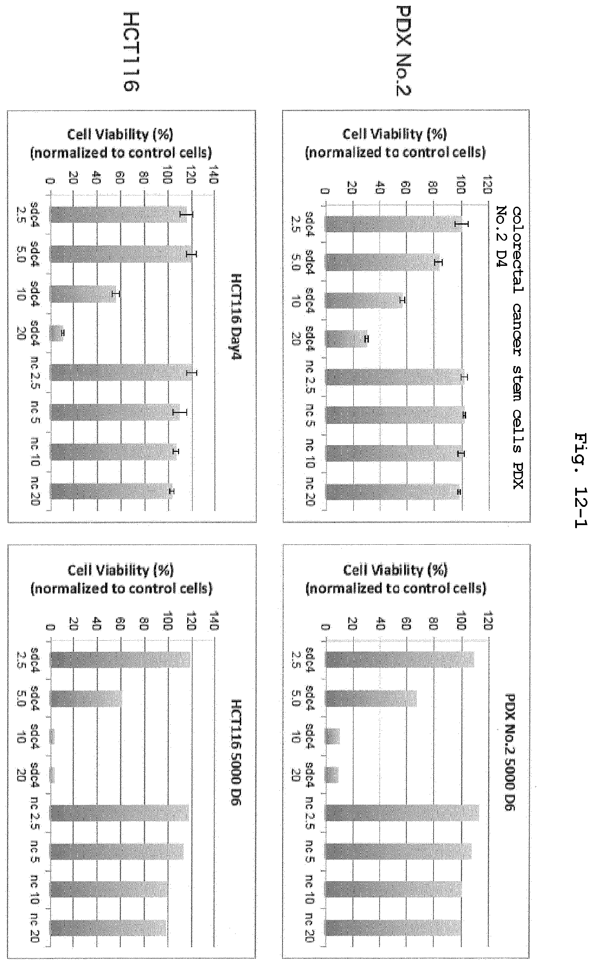

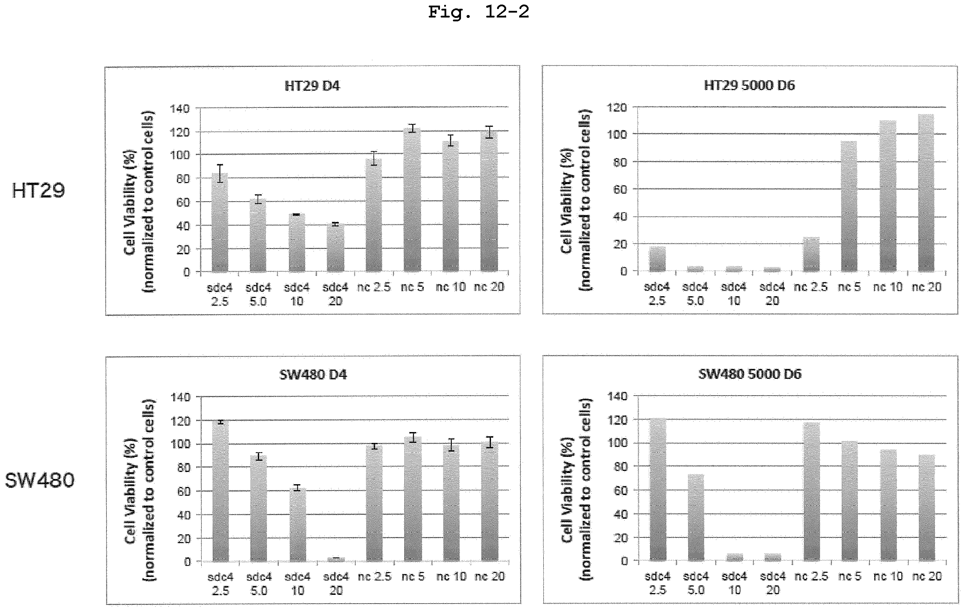

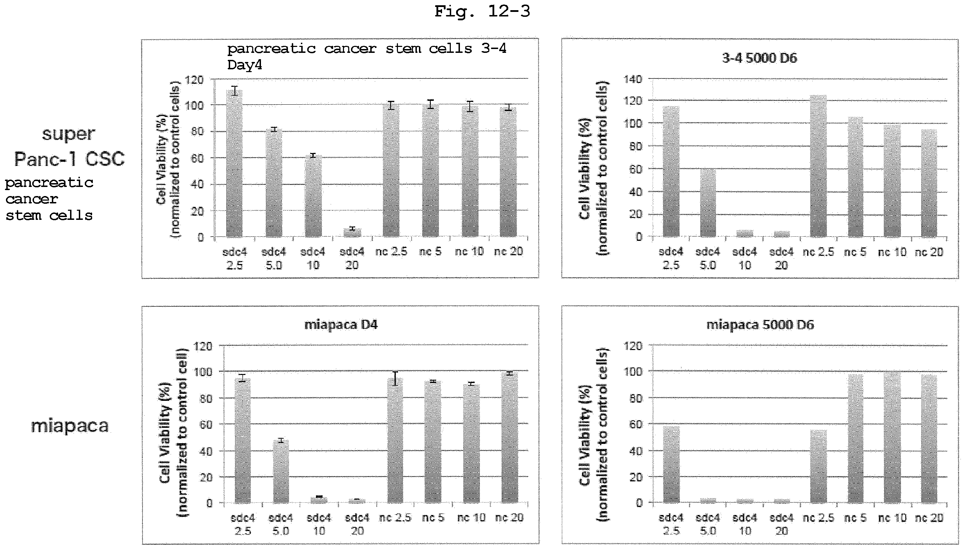

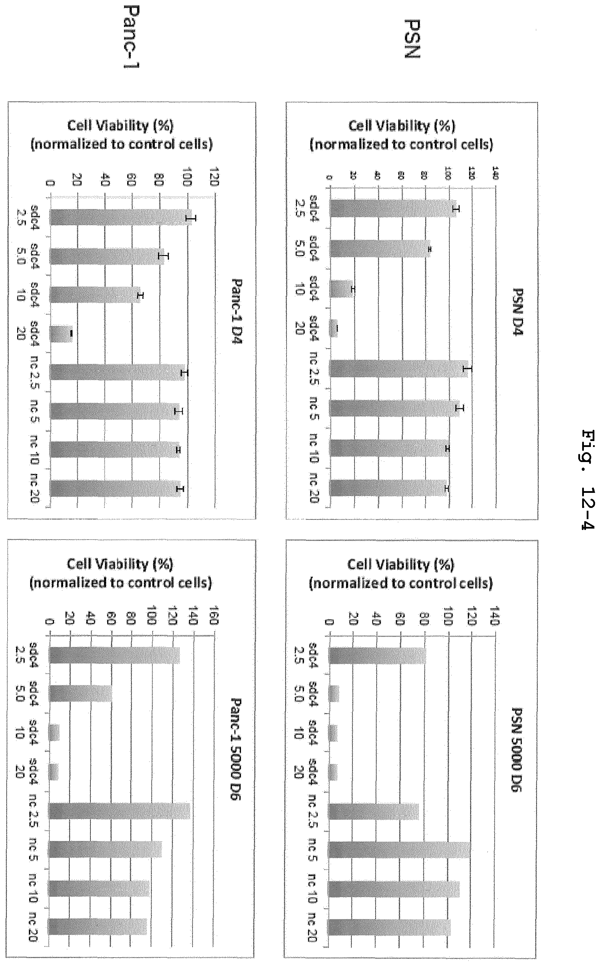

The antitumor cell effect on cell line PDX No.2 derived from PDX model of cancer tissue excised from No.2 H16-2490 colorectal cancer patient, and colorectal cancer cell lines HCT116, HT29, SW480, and further, pancreatic cancer stem cell super Panc-1 CSC and pancreatic cancer cell lines miapaca, PSN, Panc-1 was verified. 5000 cells/well were sorted by FACS into each of the 96 wells and allowed to stand overnight. SDC4 antibody which is a polyclonal antibody manufactured by IBL or IgG antibody (as NC) was added to achieve 2.5, 5, 10 and 20 ug/ml, and the cell viability was measured by WST8 on Day4 and Day6. As a result, the anti-SDC4 antibody showed a remarkable antitumor cell effect on any cancer cell line (Fig. 12-1 to Fig. 12-4) .

### (2) Toxicity of SDC4 antibody to normal cell

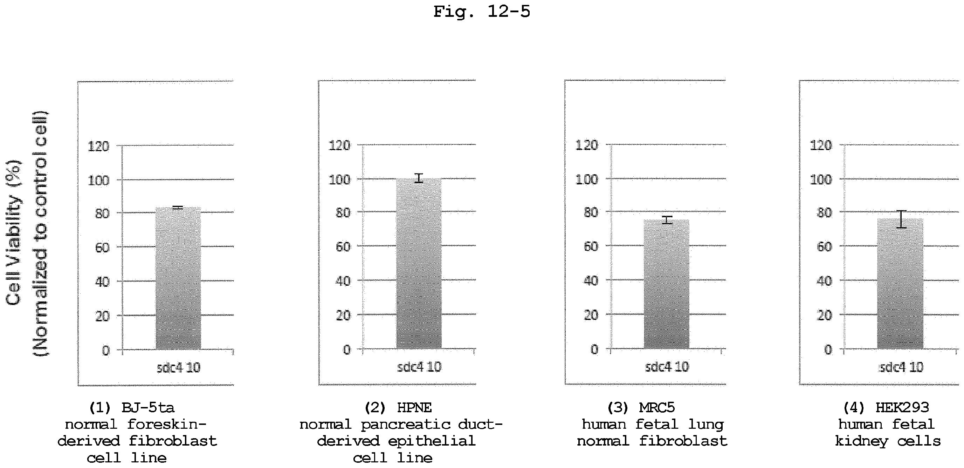

BJ-5ta normal foreskin-derived fibroblast line, HPNE normal pancreatic duct-derived epithelial cell, MRC5 human fetal lung normal fibroblast, and HEK293 human fetal kidney cells were sorted by FACS in each of the 96 wells at 5000 cells/well, allowed to stand overnight, SDC4 antibody which is a polyclonal antibody manufactured by IBL was added to achieve 10 ug/ml, and the cell viability was measured by WST8 on Day4. As a result, the anti-SDC4 antibody was relatively non-toxic to any normal cells (Fig. 12-5).

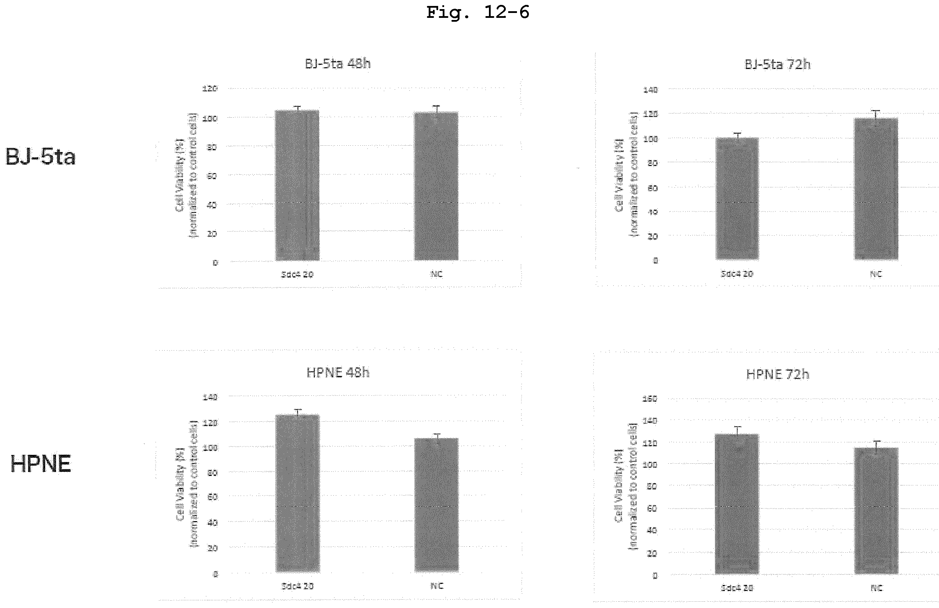

In addition, BJ-5ta normal foreskin-derived fibroblast line, and HPNE normal pancreatic duct-derived epithelial cells were plated on 96 wells to achieve 5000 cells/well, allowed to stand overnight, SDC4 antibody which is a polyclonal antibody manufactured by IBL or IgG antibody (as NC) was added to achieve 20 ug/ml, and the cell viability was measured by WST8 at 48 hr and 72 hr. As a result, the anti-SDC4 antibody was relatively non-toxic to any normal cells (Fig. 12-6).

### (3) Antitumor effect of anti-SDC4 antibody on mouse subcutaneous solid tumor model

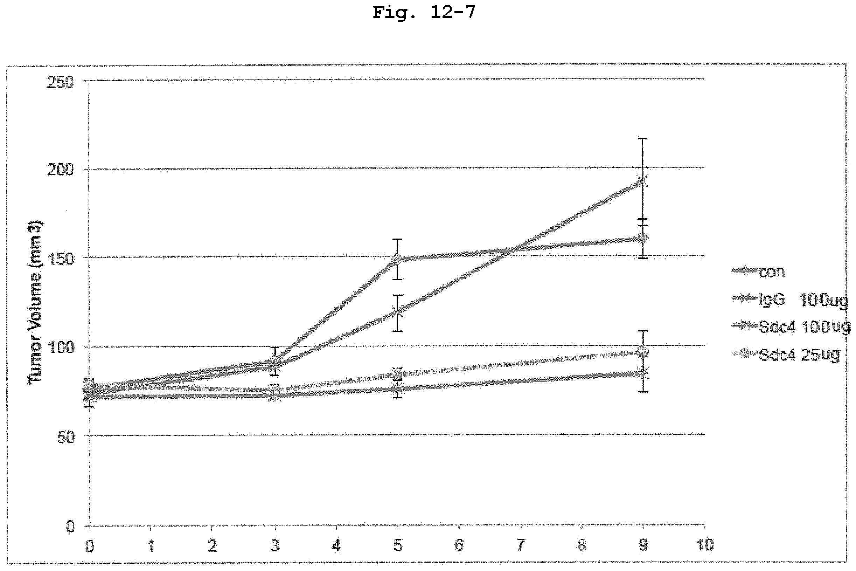

Super Panc-1 CSC pancreatic cancer stem cells were subcutaneously transplanted to a nude mouse at 3×10⁶ cells/Tumor. When the volume reached about 70 mm³, SDC4 antibody, which is a polyclonal antibody manufactured by IBL, or IgG antibody was intraperitoneally injected to the mouse once every two days with 1 ml at 100 ug/ml or 25 ug/ml. As a result, a remarkable antitumor effect was found in the SDC4 100 ug and SDC4 25 ug administration groups as compared with the untreated group (control) and the negative control administration group (IgG 100 ug). On Day5 and Day9, the volume of the tumor was significantly small in the SDC4 administration group as compared with the control and NC (Fig. 12-7) .

### Example 5 Antitumor effect of treatment targeting SDC4 on mesothelioma

### (1) Antitumor effect of siRNA targeting SDC4 on mesothelioma cell line

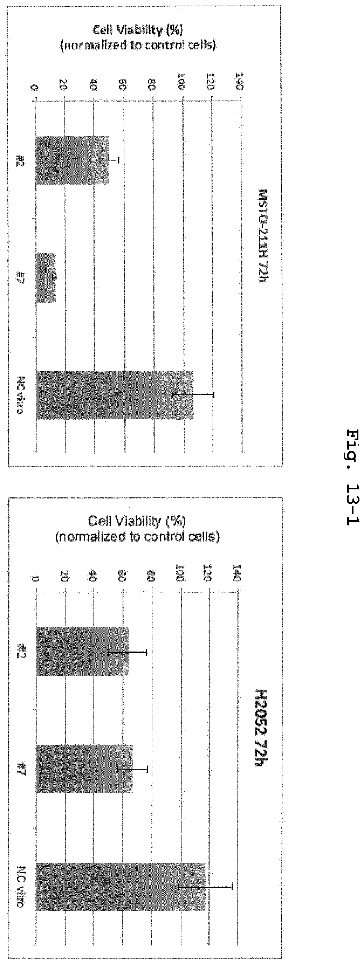

According to the Cancer Cell Line Encyclopedia (CCLE) database, the SDC4 mRNA expression level of mesothelioma is as high as the 3^{rd} place overall. Thus, using commercially available siRNA#2 targeting SDC4, siRNA#7 uniquely designed by the present inventors, and the negative control siRNA NC, the antitumor cell effect on two types of mesothelioma cell lines MSTO-211H and H2052 was verified. 5000 cells/well were sorted by FACS into each of the 96 wells and allowed to stand overnight, siRNA 10 pmol/well was introduced by lipofectamine2000, and after 72 hr, the cell viability was measured. As a result, siRNA targeting SDC4 showed a remarkable antitumor cell effect (Fig. 13-1).

### (2) Antitumor effect of anti-SDC4 antibody on mesothelioma cell line and cancer stem cell fraction thereof

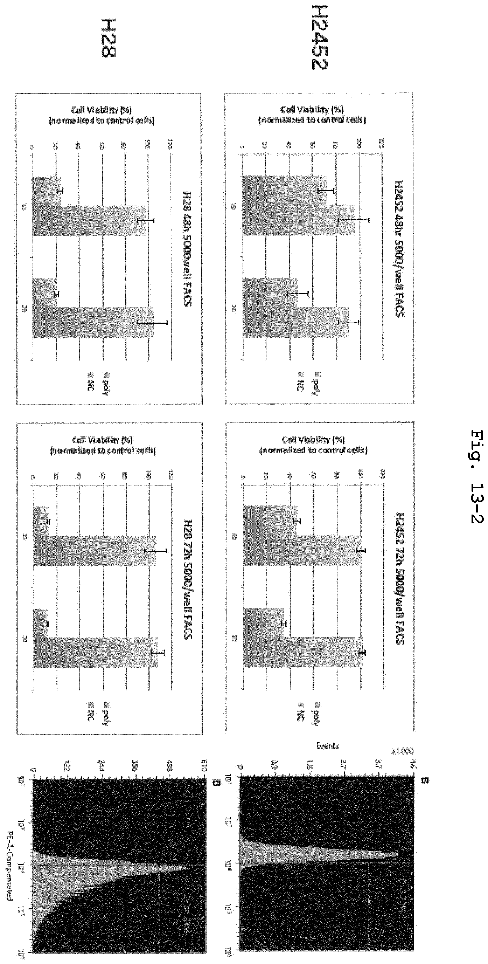

The antitumor cell effect on mesothelioma cell lines H2452 and H28 was verified. 5000 cells/well were sorted by FACS into each of the 96 wells and allowed to stand overnight. SDC4 antibody which is a polyclonal antibody manufactured by IBL (poly) or IgG antibody (NC) was added to achieve 10 and 20 ug/ml, and the cell viability was measured by WST8 48 hr and 72 hr later. As a result, the anti-SDC4 antibody showed a remarkable antitumor cell effect (Fig. 13-2).

CD24 is known as a cancer stem cell marker for mesothelioma, and it has been reported in literatures that CD24-positive cell has strong properties as a cancer stem cell. The CD24 positive rate of H2452 is 3.7%, whereas the CD24 positive rate of H28 is as high as 81.8%. As compared with H2452, the SDC4 antibody showed an earlier (48 hr than 72 hr) and more remarkable antitumor cell effect on H28. Therefore, it was found that the anti-SDC4 antibody has a particularly strong antitumor cell effect on mesothelioma cancer stem cells.

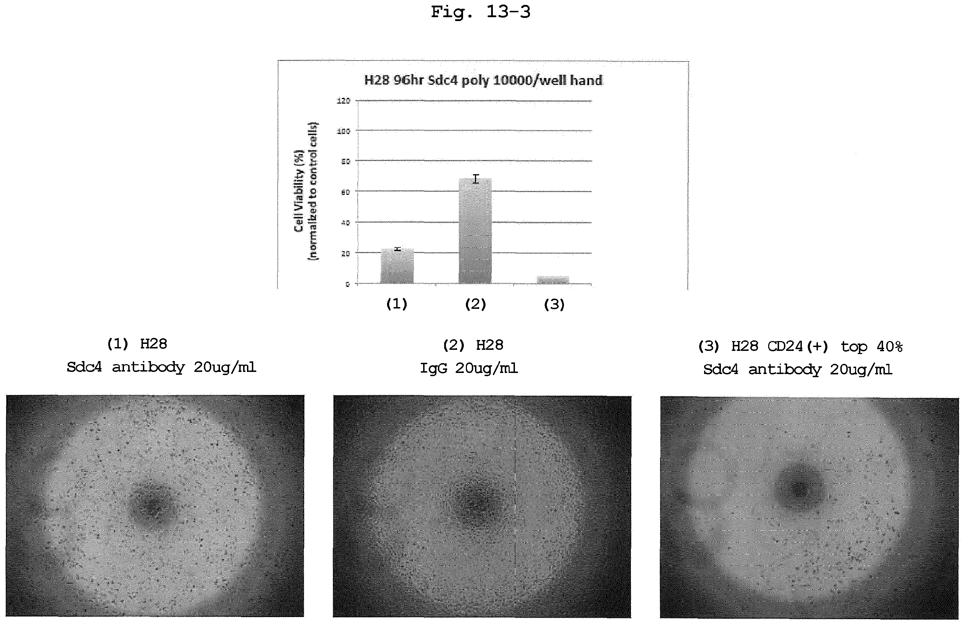

Thus, mesothelioma cell line H28 was plated on 96 wells to achieve 10000 cells/well, top 40% of CD24-positive cells were further sorted by FACS to achieve 10000 cells in 96 wells, and allowed to stand overnight. SDC4 antibody which is a polyclonal antibody manufactured by IBL (poly) or IgG antibody (NC) was added to achieve 20 ug/ml, and the cell viability was measured by WST8 96 hr later. As a result, the anti-SDC4 antibody not only showed a remarkable antitumor cell effect on unfractionated H28 but also a remarkable antitumor cell effect on highly CD24-positive H28 cell population (Fig. 13-3).

### [Industrial Applicability]

Since SDC4 is a cancer stem cell marker superior to known cancer stem cell markers, detection and sorting of cancer stem cell by using the expression of SDC4 as an index is useful for identification of cancer stem cells and research use thereof.

Furthermore, since SDC4 is not a mere marker, but becomes a target in the treatment of cancer stem cells, a medicament targeting SDC4 such as nucleic acid and the like can be utilized for elimination of cancer stem cells and a permanent cure of cancer, and, in turn, is extremely useful.

## Claims

1. A cancer stem cell elimination agent comprising a substance that suppresses an expression or function of SDC4.

2. The agent according to claim 1, wherein the substance that suppresses the expression of SDC4 is
(a) a nucleic acid having RNAi activity against a transcription product of SDC4 gene, or a precursor thereof,
(b) an antisense nucleic acid against a transcription product of SDC4 gene, or
(c) a ribozyme nucleic acid against a transcription product of SDC4 gene.

3. The agent according to claim 2, wherein the substance that suppresses the expression of SDC4 is a nucleic acid comprising a nucleotide sequence complementary to a sequence consisting of at least 15 consecutive nucleotides in the region indicated by nucleotide numbers 41 - 637 in the nucleotide sequence shown in SEQ ID NO: 1, or a precursor thereof.

4. The agent according to claim 2, wherein the substance that suppresses the expression of SDC4 is a nucleic acid comprising a nucleotide sequence complementary to a sequence consisting of at least 15 consecutive nucleotides in the region indicated by nucleotide numbers 475 - 606 in the nucleotide sequence shown in SEQ ID NO: 1, or a precursor thereof.

5. The agent according to claim 3 or 4, wherein the agent is siRNA.

6. The agent according to claim 1, wherein the substance that suppresses the function of SDC4 is
(a) an antibody against SDC4, or
(b) an antagonist against SDC4.

7. The agent according to any one of claims 1 to 6, wherein the agent is in combination with other antitumor drug.

8. The agent according to claim 7, wherein said other antitumor drug is one or more kinds selected from a chemotherapy drug, a Wnt inhibitor and an mTOR inhibitor.

9. A method for detecting or sorting a cancer stem cell in a cancer cell population, comprising using an expression of SDC4 as an index.

10. A reagent for detecting a cancer stem cell, comprising an antibody against SDC4.
